# EUROPEAN PATENT SPECIFICATION

(11) **EP 1 313 864 B1**
(45) Date of publication and mention of the grant of the patent: **07.12.2011**
(21) Application number: 01968285.5
(22) Date of filing: 30.08.2001
(51) Int. Cl.: C12N 15/64, C12N 15/65, A61K 48/00, A61P 31/04

(54) **ANTI-MICROBIAL AGENTS**
ANTIMIKROBIELLE WIRKSTOFFE
AGENTS ANTIMICROBIENS

(30) Priority: 30.08.2000 US 651290
(43) Date of publication of application: 28.05.2003
(73) Proprietor: WISCONSIN ALUMNI RESEARCH FOUNDATION, Madison, WI 53705 (US)
(72) Inventor: FILUTOWICZ, Marcin, S., Madison, WI 53711-2638 (US)
(74) Representative: Cripps, Joanna Elizabeth
(86) International application number: PCT/US2001/027028
(87) International publication number: WO 2002/018605

(56) References cited:
- DIAZ E ET AL: "UNIVERSAL BARRIER TO LATERAL SPREAD OF SPECIFIC GENES AMONG MICROORGANISMS" MOLECULAR MICROBIOLOGY, BLACKWELL SCIENTIFIC, OXFORD, GB, vol. 13, no. 5, 1994, pages 855-861, XP000579401 ISSN: 0950-382X
- BLASINA ALESSANDRA ET AL: "Copy-up mutants of the plasmid RK2 replication initiation protein are defective in coupling RK2 replication origins." PROCEEDINGS OF THE NATIONAL ACADEMY OF SCIENCES OF THE UNITED STATES, vol. 93, no. 8, 1996, pages 3559-3564, XP001077749 1996 ISSN: 0027-8424

## Description

### FIELD OF THE INVENTION

The present invention relates to the field of bacteriology. In particular, the invention relates to novel antimicrobial agents comprising transmissible plasmids that kill targeted recipient bacteria, but are not harmful to donor bacteria.

### BACKGROUND OF THE INVENTION

As the use of conventional pharmaceutical antibiotics (herein referred to as antibiotics) increases for medical, veterinary and agricultural purposes, the increasing emergence of antibiotic-resistant strains of pathogenic bacteria is an unwelcome consequence. This has become of major concern inasmuch as drug resistance of bacterial pathogens is presently the major cause of failure in the treatment of infectious diseases. Indeed, people now die of certain bacterial infections that previously could have been easily treated with existing antibiotics. Such infections include, for instance, *Staphylococcus pneumoniae,* causing meningitis; *Enterobacter sp.,* causing pneumonia; *Enterococcus sp.,* causing endocarditis, and *Mycobacterium tuberculosis,* causing tuberculosis.

The emergence of single- or multi-drug resistant bacteria results from a gene mobilization that responds quickly to the strong selective pressure that is a consequence of antibiotic uses. Over the last several decades, the increasingly frequent usage of antibiotics has acted in concert with spontaneous mutations arising in the bacterial gene pool to produce antibiotic resistance in certain strains. This gene pool is continually utilized by previously sensitive strains capable of accessing it by various means including the transfer of extrachromosomal elements (plasmids) by conjugation. As a result, single- and multi-drug resistance genes are commonly found in a large variety of bacterial plasmids.

Presently there is no known method by which to avoid the selection of antibiotic resistant bacterial mutants that arise as a result of the many standard applications of antibiotics in the modem world. Accordingly, a need exists to develop alternative strategies of antibacterial treatment.

Interest in the use of bacteriophages to treat infectious bacterial diseases developed early in the twentieth century, and has undergone a resurgence in recent years. For instance, bacteriophages have been shown effective in the treatment of certain pathogenic *E*. *coli* species in laboratory and farm animals, and have been proposed as a viable alternative to the use of antibiotics (Smith & Huggins, J. Gen. Microbiol. 128: 307-318, 1981; Smith & Huggins, J. Gen. Microbiol. 129: 2659-2675, 1983; Smith et al., J. Gen. Microbiol. 133: 1111-1126, 1986; Kuvda et al., Appl. Env. Microbiol. 65: 3767-3773, 1999). However, the use of bacteriophages as antimicrobial agents has certain limitations. First, the relationship between a phage and its host bacterial cell is typically very specific, such that a broad host-range phage agent generally is unavailable. Second, the specificity of interaction usually arises at the point of the recognition and binding of phage to the host cell. This often occurs through the expression of surface receptors on the host cell to which a phage specifically binds. Inasmuch as such receptors are usually encoded by a single gene, mutations in the host bacterial cell to alter the surface receptor, thereby escaping detection by the phage, can occur with a frequency equivalent to or higher than, the mutation rate to acquire antibiotic resistance. As a result, if phage were utilized as commonly as antibiotics, resistance of pathogenic bacteria to phages could become as common a problem as antibiotic resistance.

Another approach to controlling pathogenic bacteria has been proposed, which relies on using molecular biological techniques to prevent the expression of antibiotic resistance genes in pathogenic bacteria (U.S. Patent No. 5,976,864 to Altman et al.). In this method, a nucleic acid construct encoding an "eternal guide sequence" specific for the targeted antibiotic resistance gene is introduced into the pathogenic bacterial cells. The sequence is expressed, hybridizes with messenger RNA (mRNA) encoding the antibiotic resistance gene product, and renders such mRNA sensitive to cleavage by the enzyme RNAse P. Such a system also has limited utility, since it is targeted to specific antibiotic resistance genes. While the system may be effective in overcoming resistance based on expression of those specific genes, continued use of the antibiotics places selective pressure on the bacteria to mutate other genes and develop resistance to the antibiotic by another mechanism.

It is clear from the foregoing discussion that current alternatives to antibiotic use are limited and suffer many of the same drawbacks as antibiotic use itself. Thus, a need exists for a method of controlling unwanted bacteria that is flexible in range and that cannot be overcome by the bacteria by a single or small number of mutations.

### SUMMARY OF THE INVENTION

The present invention provides pharmaceutical preparations comprising novel antibacterial agents that are efficiently transferred to bacteria, *e.g*., pathogenic bacteria, that have a flexible range, and to which the target bacteria have difficulty developing resistance. These antibacterial agents offer an effective alternative to the use of conventional antibiotics. This invention relies on a universal property of conjugative systems whereby plasmid-encoded information, even that encoding self-destruction, will be expressed upon transfer to a recipient cell. That property was used to engineer a broad-host lethal system to limit the lateral spread of cloned genes (Diaz et al., 1994, Mol. Microbiol. 13, 855-361).

According to one aspect of the invention there is provided a pharmaceutical preparation for use in treatment of a subject by reducing or eliminating a target population of bacteria in the subject, comprising an antimicrobial agent, which comprises a donor bacterial cell harboring at least one transmissible plasmid comprising:
a) an origin of replication for synthesizing the plasmid in the donor bacterial cell, wherein initiation of replication at the origin is negatively controlled by a plasmid replication repressor, wherein in the absence of the plasmid replication repressor, the transmissible plasmid undergoes runaway replication;
b) an origin of transfer from which conjugative transfer of the transmissible plasmid initiates from the donor bacterial cell to at least one recipient cell; and, optionally,
c) at least one selectable marker gene;
   wherein the donor bacteriaL cell further comprises on its chromosome, on the transmissible plasmid or both, one or more transfer genes conferring upon the donor bacteriaL cell the ability to conjugatively transfer the transmissible plasmid to the recipient cell, and wherein the donor bacteriaL cell produces the plasmid replication repressor, and further wherein the at least one recipient cell does not produce the plasmid replication repressor, thereby enabling the transmissible plasmid to undergo runaway replication in the recipient cell to kill the recipient cell.

According to another aspect of the invention there is provided a pharmaceutical preparation for use in treatment of a subject by reducing or eliminating a target population of bacteria in the subject, comprising an antimicrobial agent, which comprises a donor bacteria cell harboring at least one transmissible plasmid comprising:
a) an origin of replication for synthesizing the plasmid in the donor bacteriaL cell;
b) an origin of transfer from which conjugative transfer of the transmissible plasmid initiates from the donor bacteriaL cell to at least one recipient cell;
c) at least one killer gene that, upon expression in a bacterial cell, produces a product that kills the cell; and, optionally,
d) at least one selectable marker gene;
   wherein the donor bacteriaL cell further comprises on its chromosome, on the transmissible plasmid or both, one or more transfer genes conferring upon the donor bacteriaL cell the ability to conjugatively transfer the transmissible plasmid to the recipient cell, and wherein the donor bacteriaL cell is modified so as to be unaffected by the product of the killer gene, and further wherein the at least one recipient cell has not been modified so as to be unaffected by the product of the killer gene.

Also described herein are methods of treating a subject for a bacterial infection, which comprises administering to the subject one of the aforementioned antibacterial agents. A mode of administration is selectedsuch that the donor cells of the antibacterial agent come into conjugative proximity to the unwanted recipientcells, such that the transmissible plasmids of the donor cells are conjugatively transferred from the donors to the unwanted recipient cells. In some embodiments, the transferred plasmidundergoes unchecked replication. In other embodiments, at least one killer gene is expressed to produce a gene product that is detrimental or lethal to the unwanted recipientcells.

The present invention also provides pharmaceutical preparations for treating a patient for a bacterial infection. These preparations comprise one of the aforementioned antibacterial agents, formulated for a pre-determined route of administration to the patient.

Also described herein are methods of using the antibacterial agents of the invention in agricultural, veterinary, environmental and food maintenance applications. In these methods, the antibacterial agents of the invention are applied to (1) plant surfaces to reduce or prevent bacterial plant disease or spoilage, (2) food surfaces to reduce or prevent post harvest spoilage of vegetables, meat or fish, (3) animal feed to reduce the bio-burden. Other similar applications are also provided.

Other features and advantages of the present invention will be understood by reference to the drawings, detailed description and examples that follow.

### BRIEF DESCRIPTION OF THE DRAWINGS

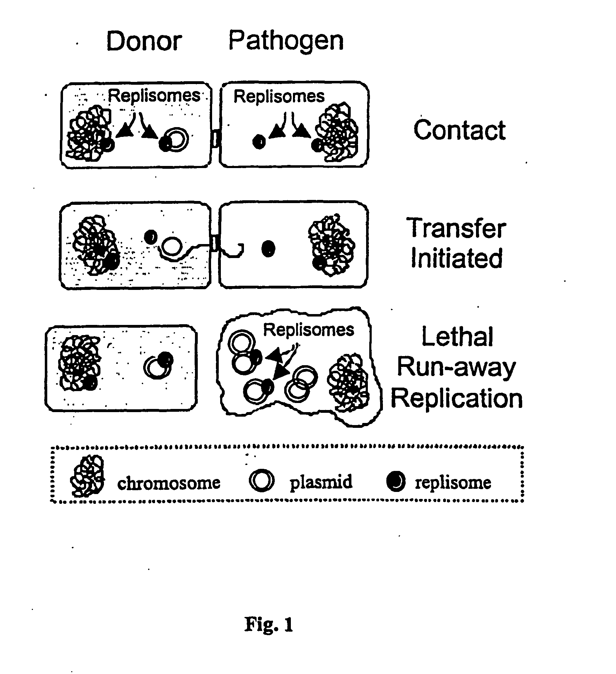

**Fig. 1****.** Schematic diagram showing a process of killing bacteria by conjugative transfer of plasmids that engage in runaway replication in the recipient cells.
**Fig. 2A****.** Schematic diagram of a non-self-transmissible, runaway replication plasmid system using a helper plasmid and a transmissible runaway replication plasmid.

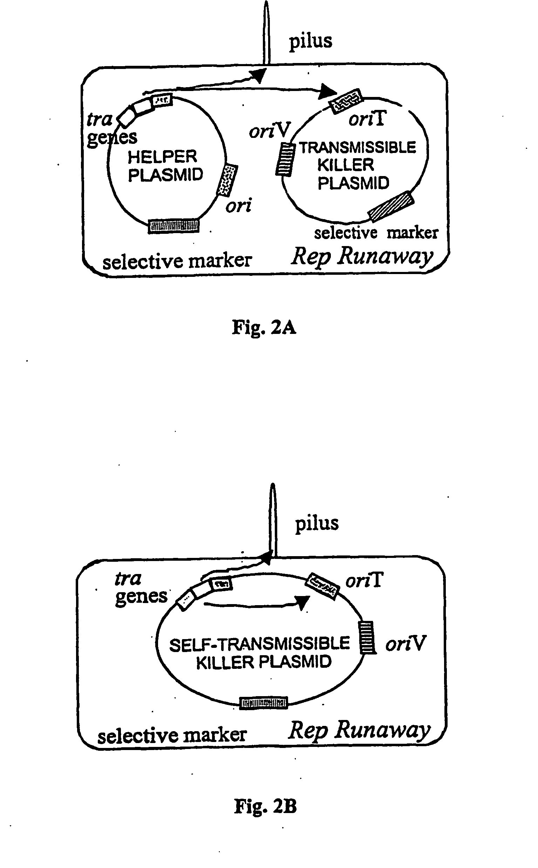

**Fig. 2****B.** Schematic diagram of a self-transmissible, runaway replication plasmid system.

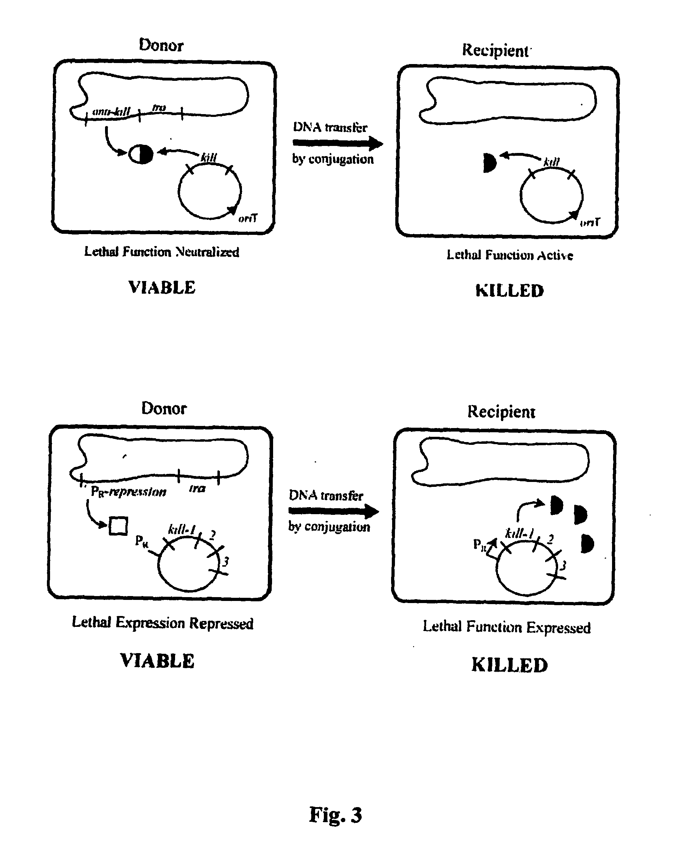

**Fig. 3****.** Schematic diagrams showing a "Trojan-Horse"-like process of killing bacteria by conjugative transfer of plasmids that encode a kill product that is neutralized by an anti-kill product in the donor but is not neutralized in the recipient that lacks anti-kill gene as part of its chromosome (top). Bottom diagram represents a general scenario of process of killing bacteria by conjugatively transferred plasmid that contains a synthetically assembled operon that encodes one or more kill products. Expression of the operon is repressed in the donor but not in the recipient. The kill gene products can be either natural or man-made peptides or RNA.

### DETAILED DESCRIPTION OF THE INVENTION

The present invention provides novel antibacterial strategies that utilize the highly efficient bacterial conjugation system to transfer a "killer" plasmid from a donor cell that is engineered to be resistant to the killer plasmid, to a target bacterial cell that is not.

In one aspect of the invention, the "killer plasmid" is one that undergoes runaway replication in the recipient cells, ultimately killing the cells. The basic principles underlying the mechanism by which runaway plasmid replication kills cells are outlined below.

Plasmids are generally dispensable DNA molecules that are stably maintained in bacterial populations. Plasmids replicate extra-chromosomally inside the bacterium and can transfer their DNA from one cell to another by a variety of mechanisms. DNA sequences controlling extra-chromosomal replication (*ori*) and transfer (*tra*) are distinct from one another; i.e., a replication sequence generally does not control plasmid transfer, or vice-versa. Replication and transfer are both complex molecular processes that make use of both plasmid- and host-encoded functions.

Bacterial conjugation is the unidirectional and horizontal transmission of genetic information from one bacterium to another. The genetic material transferred may be a plasmid or it may be part of a chromosome. Bacterial cells possessing a conjugative plasmid contain a surface structure (the sex pilus) that is involved in the coupling of donor and recipient cells, and the transfer of the genetic information. Conjugation involves contact between cells, and the transfer of genetic traits can be mediated by many plasmids.

Among all natural transfer mechanisms, conjugation is the most efficient. For example, F plasmid of *E. coli,* pCF10 plasmid of *Enterococcus faecalis* and pXO16 plasmid of *Bacillus thuringiensis* employ different mechanisms for the establishment of mating pairs, the sizes of mating aggregates are different, and they have different host ranges within gram-negative (F) as well as gram-positive (pCF10 and pXO16) bacteria. Their plasmid sizes are also different; 54, 100 and 200 kb, respectively. Remarkably, however, those conjugation systems have very important characteristics in common: they are able to sustain conjugative transfer in liquid medium and transfer efficiencies close to 100% are often reached in a very short time (Dunny et al., 1982, J. Bacteriol. 151, 855-859; Andrup, et al.,. 1998, Plasmid 40, 30-43; Andrup L, and Andersen, K., 1999), Microbiology 145, 2001-2009; and Jansen et al., 1996, Curr. Microbiol. 33, 228-236. Thus, the conjugative process permits the protection of plasmid DNA against environmental nucleases, and the very efficient delivery of plasmid DNA into a recipient cell.

Conjugation functions are plasmid encoded. Numerous conjugative plasmids (and transposons) are known, which can transfer associated genes within one species (narrow host range) or between many species (broad host range). Transmissible plasmids have been reported in numerous Gram-positive genera, including but not limited to pathogenic strains of *Streptococcus, Staphylococcus, Bacillus, Clostridium* and *Nocardia.* The early stages of conjugation generally differ in Gram-negative and Gram-positive bacteria. The role of some of the transfer genes in conjugative plasmids from Gram-negative bacteria is to provide pilus-mediated cell-to-cell contact, formation of a conjugation pore and related morphological functions. The pili do not appear to be involved in initiating conjugation in Gram-positive bacteria. The feature best understood in the *Enterococci* is the involvement of pheromones. Pheromones are hydrophobic polypeptides of 7-8 amino acids produced by potential recipient cells. Pheromones invite attention of potential donor cells containing conjugative plasmids. PAD1 is one of the best studied pheromone-induced plasmids which can replicate in 50 different bacterial hosts in addition to *Enterococcus faecelis* strains from which it was initially isolated (Clewell D.B. 1999. Sex pheromone systems in Enterococci, In: Cell-Cell Signaling in Bacteria, Ed. G.M. Dunny, S.C. Winans; ASM, Washington D.C. pp 47-65). Moreover, conjugation can occur between genera as widely diverse as anaerobes and aerobes.

Naturally occurring plasmids are present within host cells at a characteristic concentration (referred to herein as a particular plasmid "copy number"). Of great significance to the present invention is the fact that plasmid copy number is negatively controlled Helinski et al., 1996 (In Escherichia coli and Salmonella Cellular and Molecular Biology, Vol. 2 (ed. F. Neidhardt, et al., 2295-2324, ASM Press, Washington D.C.). Thus, mutations that destroy the elements of the negative control cause an over-replication phenotype that manifests itself by an increase in the plasmid copy number ("copy-up" phenotype). In extreme cases of copy-up mutations, plasmid replication is completely unchecked due to the loss of copy control mechanisms. This is referred to as "runaway plasmid replication" or simply "runaway replication."

Runaway plasmid replication is lethal for the host cell. This is because, although the plasmid encodes the replication (Rep) protein that controls its copy number, all other replication proteins are encoded by chromosomal genes. These chromosomally encoded proteins assemble into a complex called a replisome. A typical bacterial cell possesses a small, fixed number of replisomes. Because both the chromosome and the plasmids require the same replisomes for DNA synthesis, an excess of plasmids acts like a trap to occupy all of the replisomes within the cell. This results in the inability of the chromosome to replicate, ultimately leading to the death of the cell.

The use of runaway replication plasmids as a means to kill recipient cells has a number of advantages over conventional antibiotic methodologies. One significant advantage is that, since the entire host replication machinery is targeted, multiple mutations would be required to avoid death by elevating the expression or activity of the replisome sub-assemblies. For instance, mutations in ten genes would be required just to increase the amount or activity of DNA polymerase III holoenzyme (composed of ten different subunits), and this polymerase is just one of the replisome sub-assemblies. Thus, there is little or no chance of a bacterium acquiring resistance to being killed by over-replicating plasmids. In contrast, conventional antibiotics typically inhibit only a single enzymatic activity that is essential for the survival of a cell. A single-target strategy and the relatively high spontaneous mutation frequency for one gene (10⁻⁶ to 10⁻⁸) unavoidably leads to the quick acquisition of resistance to such drugs.

Because runaway replication mutations are lethal to the host cell, the donor cells that maintain such plasmids are generally engineered so that replication of the plasmid is controlled *e.g.*, by providing a wild-type Rep protein to the host cell. In some embodiments, this is accomplished by providing a Rep gene on another plasmid. In other embodiments, a *rep* gene is provided by integration into the bacterial chromosome of a donor cell using standard homologous recombination techniques.

In some embodiments, the antimicrobial strategy of the present invention comprises:
(1) a plasmid that, alone or with the assistance of a helper plasmid, comprises the genes necessary to effect conjugative transfer of the plasmid from a donor cell to a recipient cell, wherein replication of the plasmid is controlled, *e.g.*, repressed, by a reversible mechanism, such as control by a product of a gene that can be de-activated (*e.g.*, via mutation) so as to release the control on plasmid replication (referred to herein as a "runaway replication plasmid");
(2) a source of conjugative transfer genes (*e.g.*, on the runaway replication plasmid, or on a separate "helper" plasmid); and
(3) a donor cell for maintaining the runaway replication plasmid in a replication-suppressed state, so as not to be killed by the plasmid.

A number of plasmids have been well characterized, and can serve as subjects for mutagenesis to create runaway mutants, which may be used in embodiments of the present invention. Such mutant plasmids contain, or can be easily modified to contain all components needed for conjugative transfer from donor to recipient cells but are defective in their replicative repressor (Rep) function. Examples of such mutants, both broad-range and narrow-range, are known in the art (Haugan et al., Plasmid 33: 27-39, 1995; Molin et al., J. Bacteriol.143: 1046-1048, 1980; Toukdarian & Helinski, Gene 223: 205-211, 1998). A particularly preferred plasmid of this type is a mutant of plasmid R6K, as described in detail in Examples 1 and 2. Other examples include, but are not limited to, RK2, pCU1, p15A, pIP501, pAMβ1 and pCRG1600.

As an alternative to the use of mutants, it may sometimes be preferable to use various components of conjugative plasmids whose features are well understood, to create plasmids having all necessary features. Such runaway replication plasmids or helper plasmids may include (1) an origin of replication (*e.g*., *ori*V as decribed herein), a sequence from which replication of the plasmid originates and the sequence that may be negatively regulated by a Rep protein; (2) an origin of transfer (*e.g., ori*T as described herein), a sequence from which a conjugal plasmid transfer originates; (3) transfer (*tra*) genes to effect conjugation; and (4) a screenable/selectable marker gene. The donor cell containing the runaway replication plasmid is engineered to contain a functional repressor (Rep) of replication at *ori*V, thereby controlling replication of the runaway replication plasmid while it is still in the donor.

Non-self-transmissible plasmid systems and self-transmissible plasmid systems are contemplated. Examples of these are shown schematically in Figs. 2A and 2B. The systems diagrammed here and described below are provided as examples of the systems of the present invention and are not be construed as limiting the components or sources of components assembled to effect the methods and compositions of the invention. For example, where particular genes or genetic elements providing particular functions are named (*e.g.*, the *oriV* origin of replication, the *oriT* origin of transfer), it is contemplated that other genes or genetic elements providing equivalent functions or functional combinations may be used.

In some embodiments of non-self-transmissible systems (*e.g*., as shown in Fig. 2A), the runaway replication plasmid contains an *ori*T, an *ori*V and a selectable marker gene. In some embodiments, a helper plasmid contains the additional *tra* genes, along with its own origin of replication and a selective marker. Thus, the helper plasmid enables conjugative transfer of the runaway replication plasmid, but is itself confined to the donor cell due to its lack of an *oriT.* In other embodiments, the *tra* genes are integrated into the chromosome of the donor cell. Since the runaway replication plasmid lacks the necessary *tra* genes to convert the recipient cell into a donor cell, the cycle of conjugation ends with the original recipient cell. It cannot transfer its runaway replication plasmid to a second recipient before it dies.

In some embodiments of self-transmissible systems (*e.g.*, as shown in Fig. 2B), the runaway replication plasmid contains an *ori*T, an *ori*V and a selectable marker gene. It also contains the additional *tra* genes needed for conjugative transfer. Thus, unlike the non-self-transmissible plasmid described above, once this plasmid has been transmitted from the original donor to a first recipient, it is capable of transmitting itself again to subsequent recipients before the first recipient cell is killed by runaway replication. A plasmid of this type has the capability of much faster dissemination among recipient cells than the non-self-transmissible type, resulting in faster and more widespread killing of those cells.

In either the self-transmissible or the self-non-transmissible system, the donor cells generally comprise a means of controlling plasmid replication. In some embodiments, the control comprises a gene encoding a repressor of plasmid replication. For example, the Rep protein represses plasmid replication initiated at *oriV.* In some embodiments, a Rep-encoding gene is provided on a helper plasmid. In other embodiments, a Rep-encoding gene is integrated into the donor cell chromosomal DNA. Plasmid DNA comprising the Rep-encoding gene may be introduced into bacterial cells by any commonly known technique (e.g., transformation). The Rep-encoding gene can be integrated into the host genome by a site-specific recombination, according to standard methods (Li-Ch Huang, E. Wood and M. Cox; J. Bacteriol. 179: 6076-6083,1997).

A number of bacterial *ori*V's and the Rep proteins that negatively control them have been characterized. Each of these is contemplated for use in the present invention. Examples of suitable *ori*V/Rep systems for use in the invention include, but are not limited to: RK2, R6K, rts 1, p15A, RSF1010, F and P1. A wide variety of replication systems may be used in the present invention (see, e.g., U.S. Pat. No. 5,851,808). The present invention is not limited to those systems described above.

The selection of *oriV* will confer on the system its range of potential recipients for runaway replicating plasmids. In most instances it may be preferable to target a specific recipient of the runaway replication plasmid. Such instances include, but are not limited to using the conjugative runaway plasmids for combating *Enterobacteria, Enterococci, Staphylococci* and non-sporulating Gram-positive pathogens such as *Nocardia* and *Mycobacterium sp.* Examples of selective host range plasmids from which such oriV's may be obtained include, but are not limited to, P1 and F.

In instances where it is desirable to affect a wide variety of recipients, a broad range *ori*V is employed. Examples of broad range ("promiscuous") plasmids from which *ori*Vs may be obtained include, but are not limited to: R6K, RK2, p15A and RSF1010.

As used herein, the term "range" (or "host range") refers generally to parameters of both the number and diversity of different bacterial species in which a particular plasmid (natural or recombinant) can replicate. Of these two parameters, one skilled in the art would consider diversity of organisms as generally more defining of host range. For instance, if a plasmid replicates in many species of one group, e.g., *Enterobacteriaceae,* it may be considered to be of narrow host range. By comparison, if a plasmid is reported to replicate in only a few species, but those species are from phylogenetically diverse groups, that plasmid may be considered of broad host range. As discussed above, both types ofplasmids (or components thereof) will find utility in the present invention.

Conjugative transfer (*tra*) genes also have been characterized in many conjugative bacterial plasmids. The interchangeability between the gene modules conferring the ranges of hosts susceptible for conjugal transfer and vegetative replication include Gram-positive and Gram-negative species. Examples of characterized *tra* genes that are suitable for use in the present invention are the *tra* genes from: (1) F (Firth, N., Ippen-Ihler, K. and Skurray, R.A. 1996, Structure and function of F factor and mechanism of conjugation. In: Escherichia coli and Salmonella, Neidhard et al., eds., ASM Press, Washington D.C.); (2) R6K (Nunez et al., Mol. Microbiol. 24: 1157-1168, 1997); and (3) Ti (Ferrand et al., J. Bacteriol. 178: 4233-4247, 1996). Additional *tra* genes that find use with the present invention include, but are not limited to, those described in U.S. Pat. Nos. 6,180,406 and 6,251,674.

According to another aspect of the invention, the bacterial conjugation system is again utilized, this time to efficiently deliver a variety of "killer genes" to target bacterial cells. The delivery of various killer genes to bacterial cells occurs in nature, upon infection of bacteria with bacteriophages. Bacteriophages utilize a number of different mechanisms to maintain their own replication cycles, generally resulting in lysis of the host bacterial cells. Indeed, bacteriophages have been proposed and used as alternatives to antibiotics, as discussed above in the Background of the Invention. One serious drawback to the use of bacteriophages for this purpose is that they are often extremely host-specific, binding only to cell surfaces possessing specific receptors. As a result, bacteria quickly develop resistance mutations in the receptor, thereby escaping recognition by the phage. The present invention circumvents that drawback by placing one or more killer genes (*e.g*., from a phage or other source) on a conjugative plasmid. The conjugative plasmid containing the killer gene(s), like the conjugative runaway replication plasmids described above, is thereafter efficiently distributed to recipient cells, killing them shortly thereafter. Additional killing systems include, but are not limited to, those described in U.S. Pat. No. 6,277,608.

Bacteriophage kill host cells by a variety of mechanisms, many of which are encoded by a discrete set of genes in the phage genome. For instance, bacteriophage MS2 contains a gene encoding a bacterial lysis protein (Coleman et al., J. Bacteriol. 153: 1098-1100,1983). Phage T4D contains genes encoding proteins that degrade cytosine-containing DNA in bacterial host cells (Kutter and Wilberg, J. Mol. Biol. 38: 395-411, 1968). Other T4 phage encode gene products that interfere with transcription of cytosine-containing DNA (Drivdahl and Kutter, J. Bacteriol. 172: 2716-2727, 1990). Yet other T4 gene products are responsible for the disruption of the bacterial nucleoid (Bouet et al., Gene 141: 9-16,1994). Over 5000 characterized bacteriophages provide enormous reservoir of killer genes (Ackermann 2001. Arch. Vir., 146, 843-857). Such killer genes can be inserted into a conjugative plasmid such as those described above, for efficient distribution to target recipient cells.

In addition, other types of killer genes may be utilized similarly. These include naturally-occurring or synthetic genes. A nonlimiting example of a naturally-occurring gene that is suitable for use in the invention is the *hok* gene product described by Gerdes et al. (EMBO J. 5: 2023-2029, 1986). Examples of man-made nucleic acid molecules that may be used in this aspect of the invention include: (1) sequences encoding peptides with bactericidal activity and endotoxin neutralizing activity for Gram-negative bacteria as described in U.S. Patent 5,830,860; (2) sequences encoding RNA molecules with binding affinity to critical bacterial cellular targets (e.g., Chen and Gold, Biochemistry 33: 8746-8756, 1994); and (3) oligonucleotides generated by the *SELEX* method for the *in vitro* evolution of nucleic acid molecules with highly specific binding to target molecules as described in U.S. Pat. No. 5,475,096 and U.S. Pat. No. 5,270,163.

In these systems, steps may be employed to prevent death of the donor cell . For example, the death of the donor cell can be prevented by employing a synthetic promoter-operator system whose expression is prevented by the repressor made only in the donor cells (Fig. 3 bottom). In other embodiments, the toxin can be neutralized by an antitoxin made in donor but not in recipient bacteria (Fig. 3 top).

In preferred embodiments, the plasmid contains a screenable or selectable marker gene. In traditional molecular biological manipulations of recombinant bacteria, the selectable marker gene is an antibiotic resistance gene. Since the present invention is designed to avoid further spread of antibiotic resistance, an alternative selectable marker system is preferred for use in the present invention. Accordingly, though antibiotic resistance markers can be used in laboratory tests, preferred selectable markers are nutritional markers, i.e., any auxotrophic strain (e.g., Trp⁻, Leu⁻, Pro⁻) containing a plasmid that carries a complementing gene (e.g., *trp*⁺*, leu*⁺, *pro*⁺).

The donor bacterial strain for any of the above-described killer plasmids can be any one of thousands of free-living bacteria, associated with the body of warm-blooded animals, including humans and plants. Preferably, non-pathogenic bacteria that colonize the non-sterile parts of the body (e.g., skin, digestive tract, urogenital region, mouth, nasal passages, throat and upper airway, ears and eyes) are utilized as donor cells, and the methodology of the invention is used to combat bacterial infections of these parts of the body. In another embodiment, safe donors of these plasmids are developed for combating systemic infection. Examples of particularly preferred donor bacterial species include, but are not limited to: (1) non-pathogenic strains of *Escherichia coli* (*E. coli F18* and *E. coli* strain Nissle 1917), (2) various species of *Lactobacillus* (such as *L. casei, L. plantarum, L. paracasei, L. acidophilus, L. fermentum, L. zeae* and *L. gasseri),* (3) other nonpathogenic or probiotic skin-or GI colonizing bacteria such as *Lactococcus, Bifidobacteria, Eubacteria,* and (4) bacterial mini-cells, which are anucleoid cells destined to die but still capable of transferring plasmids (see; e.g., Adler et al., Proc. Natl. Acad. Sci. USA 57; 321-326, 1970; Frazer and Curtiss III, Current Topics in Microbiology and Immunology 69: 1-84, 1975; U.S. Patent No. 4,968,619 to Curtiss III).

, In some embodiments, the target recipient cells are pathogenic bacteria dispersed throughout the body, but particularly on the skin or in the digestive tract, urogenital region, mouth, nasal passages, throat and upper airways, eyes and ears. Of particular interest for targeting and eradication are pathogenic strains of *Pseudomonas aeruginosa, Escherichia coli, Staphylococcus pneumoniae* and other species, *Enterobacter spp., Enterococcus spp.* and *Mycobacterium tuberculosis.* The present invention finds use with a wide array of target organisms (e.g., pathogenic organisms), whether in therapeutic, agricultural, or other settings, including, but not limited to, those described in U.S. patents 6,271,359, 6,261,842, 6,221,582, 6,153,381, 6,106,854, and 5,627,275. Others are also discussed herein, and still others will be readily apparent to those of skill in the art.

It is clear from the foregoing discussion that numerous types of killer plasmids (e.g., runaway replication plasmids, plasmids carrying lethal phage genes, etc.) are suitable for use in the present invention. In view of this, one of skill in the art will appreciate that a single donor bacterial strain might harbor more than one type of killer plasmid (*e.g*., runaway or toxin-producing). In other embodiments, a donor cell may harbor a killer plasmid expressing multiple kill functions, as shown in Fig. 3 (bottom) or may harbor multiple killer plasmids each expressing kill function(s) independently of the other plasmids. Thus such multiple plasmid systems can contain a plurality of plasmid-encoded functions targeted to different recipient cells. Further, two or more donor bacterial strains, each containing one or more killer plasmids, may be combined for a similar multi-target effect.

The systems of the present invention find utility for treatment of humans and in a variety of veterinary, agronomic, horticultural and food processing applications. For human and veterinary use, and depending on the cell population or tissue targeted for protection, the following modes of administration of the bacteria of the invention are contemplated: topical, oral, nasal, pulmonary/bronchial (e.g., via an inhaler), ophthalmic, rectal, urogenital, subcutaneous, intraperitoneal and intravenous. The bacteria preferably are supplied as a pharmaceutical preparation, in a delivery vehicle suitable for the mode of administration selected for the patient being treated. The term "patient" or "subject" as used herein refers to humans or animals (animals being particularly useful as models for clinical efficacy of a particular donor strain).

For instance, to deliver the bacteria to the gastrointestinal tract or to the nasal passages, the preferred mode of administration is by oral ingestion or nasal aerosol, or by feeding (alone or incorporated into the subject's feed or food). In this regard, it should be noted that probiotic bacteria, such as *Lactobacillus acidophilus,* are sold as gel capsules containing a lyophilized mixture of bacterial cells and a solid support such as mannitol. When the gel capsule is ingested with liquid, the lyophilized cells are re-hydrated and become viable, colonogenic bacteria. Thus, in a similar fashion, donor bacterial cells of the present invention can be supplied as a powdered, lyophilized preparation in a gel capsule, or in bulk for sprinkling into food or beverages. The re-hydrated, viable bacterial cells will then populate and/or colonize sites throughout the upper and lower gastrointestinal system, and thereafter come into contact with the target pathogenic bacteria.

For topical applications, the bacteria may be formulated as an ointment or cream to be spread on the affected skin surface. Ointment or cream formulations are also suitable for rectal or vaginal delivery, along with other standard formulations, such as suppositories. The appropriate formulations for topical, vaginal or rectal administration are well known to medicinal chemists.

The present invention will be of particular utility for topical or mucosal administrations to treat a variety of bacterial infections or bacterially related undesirable conditions. Some representative examples of these uses include treatment of (1) conjunctivitis, caused by *Haemophilus sp.,* and corneal ulcers, caused by *Pseudomonas aeruginosa*; (2) otititis externa, caused by *Pseudomonas aeruginosa*; (3) chronic sinusitis, caused by many Gram-positive cocci and Gram-negative rods, and for general decontamination of bronchii; (4) cystic fibrosis, associated with *Pseudomonas aeruginosa*; (5) enteritis, caused by *Helicobacter pylori* (ulcers), *Escherichia coli, Salmonella typhimurium, Campylobacter* and *Shigella sp*.; (6) open wounds, both surgical and non-surgical, as a prophylactic measure for many species; (7) bums to eliminate *Pseudomonas aeruginosa* or other Gram-negative pathogens; (8) acne, caused by *Propionobacter acnes*; (9) nose and skin infections caused by methicillin resistant *Staphylococcus aureus* (MSRA); (10) body odor caused mainly by Gram-positive anaerobic bacteria (i.e., use in deodorants); (11) bacterial vaginosis associated with *Gardnerella vaginalis* and other anaerobes; and (12) gingivitis and/or tooth decay caused by various organisms.

In other embodiments, the antimicrobials of the present invention find application in the treatment of surfaces for the removal or attenuation of unwanted bacteria. For example, surfaces that may be used in invasive treatments such as surgery, catheterization and the like may be treated to prevent infection of a subject by bacterial contaminants on the surface. It is contemplated that the methods and compositions of the present invention may be used to treat numerous surfaces, objects, materials and the like (*e.g*., medical or first aid equipment, nursery and kitchen equipment and surfaces) to control bacterial contamination thereon.

Pharmaceutical preparations comprising the donor bacteria are formulated in dosage unit form for ease of administration and uniformity of dosage. Dosage unit form, as used herein, refers to a physically discrete unit of the pharmaceutical preparation appropriate for the patient undergoing treatment. Each dosage should contain a quantity of the donor bacteria calculated to produce the desired antibacterial effect in association with the selected pharmaceutical carrier. Procedures for determining the appropriate dosage unit are well known to those skilled in the art.

Dosage units may be proportionately increased or decreased based on the weight of the patient. Appropriate concentrations for achieving eradication of pathogenic bacteria in a target cell population or tissue may be determined by dosage concentration curve calculations, as known in the art.

Other uses for the donor bacteria of the invention are also contemplated. These include a variety agricultural, horticultural, environmental and food processing applications. For example, in agriculture and horticulture, various plant pathogenic bacteria may be targeted in order to minimize plant disease. One example of a plant pathogen suitable for targeting is *Erwinia amylovora,* the causal agent of fire blight. Similar strategies may be utilized to reduce or prevent wilting of cut flowers.

In veterinary or animal agriculture, the killer plasmid systems of the invention may be incorporated into animal feed (chicken, cattle) to reduce bio-burden or to eliminate certain pathogenic organisms (e.g., *Salmonella*). In other embodiments, the invention may be utilized on meat or other foods to eliminate unwanted or pathogenic bacteria (e.g., *E. coli* O157:H7 on meat, or *Proteus spp.,* one cause of "fishy odor" on seafood).

Environmental utilities comprise, for example, engineering *Bacillus thurengiensis* and one of its conjugative plasmids to deliver and conditionally express insecticidal agents (e.g., for the control of mosquitos that disseminate malaria or West Nile virus). In such applications, as well as in the agricultural and horticultural applications described above, formulation of the killer plasmids and donor bacteria as solutions, aerosols, or gel capsules are contemplated.

In preferred embodiments of the present invention, certain features are employed in the plasmids and donor cells of the invention to minimize potential risks associated with the use of DNA or genetically modified organisms in the environment. For instance, in environmentally sensitive circumstances it is preferable to utilize non-self-transmissible plasmids. Instead, the plasmids will be mobilizable by conjugative machinery but will not be self-transmissible. As discussed hereinabove, this may be accomplished in some embodiments by integrating into the host chromosome all *tra* genes whose products are necessary for the assembly of conjugative machinery. In such embodiments, killer plasmids are configured to possess only an origin of transfer (*ori*T). This feature prevents the recipient, before or even after it dies, from transferring the killer plasmid further.

Another biosafety feature comprises utilizing conjugation systems with pre-determined host-ranges. As discussed above, certain elements are known to function only in few related bacteria (narrow-host-range) and others are known to function in many unrelated bacteria (broad-host-range or promiscuous) (del Solar et al., Mol. Microbiol. 32: 661-666,1996; Zatyka and Thomas, FEMS Microbiol. Rev. 21: 291-319,1998). Also, many of those conjugation systems can function in either gram-positive or gram-negative bacteria but generally not in both (del Solar, 1996, *supra*; Zatyka and Thomas, 1998, *supra*).

Also as discussed in detail above, inadvertant proliferation of antibiotic resistance is minimized in this invention by avoiding the use of antibiotic resistance markers. In a preferred alternative approach, the gene responsible for the synthesis of an amino acid (i.e. serine) can be mutated, generating the requirement for this amino acid in the donor. Such mutant bacteria will prosper on media lacking serine provided that they contain a plasmid with the *ser* gene whose product is needed for growth. Thus, the invention contemplates the advantageous use of plasmids containing the *ser* gene or one of many other nutritional genetic markers. These markers will permit selection and maintenance of the killer plasmids in donor cells.

Another biosafety approach comprises the use of restriction-modification systems to modulate the host range of killer plasmids. Conjugation and plasmid establishment are expected to occur more frequently between taxonomically related species in which plasmid can evade restriction systems and replicate. Type II restriction endonucleases make a double-strand break within or near a specific recognition sequence of duplex DNA. Cognate modification enzymes can methylate the same sequence and protect it from cleavage. Restriction-modification systems (RM) are ubiquitous in bacteria and archaebacteria but are absent in eukaryotes. Some of RM systems are plasmid-encoded, while others are on the bacterial chromosome (Roberts and Macelis, Nucl. Acids Res. 24: 223-235, 1998). Restriction enzymes cleave foreign DNA such as viral or plasmid DNA when this DNA has not been modified by the appropriate modification enzyme. In this way, cells are protected from invasion of foreign DNA. Thus, by using a donor strain producing one or more methylases, cleavage by one or more restriction enzymes could be evaded. Site-directed mutagenesis is used to produce plasmid DNA that is either devoid of specific restriction sites or that comprises new sites, protecting or making plasmid DNA vulnerable, respectively against endonucleases. Broad-host range plasmids (eg. RP4) may evade restriction systems simply by not having many of the restriction cleavage sites that are typically present on narrow-host plasmids (Willkins et al., 1996, J. Mol. Biol 258, 447-456).

Preferred embodiments of the present invention also utilize environmentally safe bacteria as donors. For example, delivery of DNA vaccines by attenuated intracellular gram-positive and gram-negative bacteria has been reported (Dietrich et al., 2001 Vaccine 19, 2506-2512; Grillot-Courvalin et al., 1999 Current Opinion in Biotech. 10, 477-481). In addition, the donor strain can be one of thousands of harmless bacteria that colonize the non-sterile parts of the body (e.g., skin, gastrointestinal, urogenital, mouth, nasal passages, throat and upper airway systems). Examples of preferred donor bacterial species are set forth hereinabove.

In another strategy, non-dividing, non-growing donors are utilized instead of living cells. As discussed above, minicells and maxicells are well studied model systems of metabolically active but nonviable bacterial cells. Minicells lack chromosomal DNA and are generated by special mutant cells that undergo cell division without DNA replication. If the cell contains a multicopy plasmid, many of the minicells will contain plasmids. Minicells neither divide nor grow. However, minicells that possess conjugative plasmids are capable of conjugal replication and transfer of plasmid DNA to living recipient cells. (Adler et al., 1970, *supra*; Frazer and Curtiss, 1975, *supra*; U.S. Patent No. 4,968,619, *supra*).

Maxicells can be obtained from a strain of *E*. *coli* that carries mutations in the key DNA repair pathways (*rec*A*, uvr*A and *phr*)*.* Because maxicells lack so many DNA repair functions, they die upon exposure to low doses of UV. Importantly, plasmid molecules (e.g., pBR322) that do not receive an UV hit continue to replicate. Transcription and translation (plasmid-directed) can occur efficiently under such conditions (Sancar et al., J. Bacteriol. 137: 692-693, 1979), and the proteins made prior to irradiation should be sufficient to sustain conjugation. This is supported by the following two observations: i) that streptomycin-killed cells remain active donors, and ii) that transfer of conjugative plasmids can occur in the presence of antibiotics that prevent *de novo* gene expression (Heineman and Ankenbauer, 1993, J. Bacteriol. 175, 583-588;Cooper and Heineman, 2000. Plasmid 43, 171-175). Accordingly, UV-treated maxicells will be able to transfer plasmid DNA to live recipients. It should also be noted that the conservation of *rec*A and *uvr*A genes among bacteria should allow maxicells of donor strains other than *E. coli* to be obtained.

Also contemplated for use in the invention are any of the modified microorganisms that cannot function because they contain temperature-sensitive mutation(*s*) in genes that encode for essential cellular functions (e.g., cell wall, protein synthesis, RNA synthesis, as described, for example, in US patent 4,968,619, *supra*). For many approaches, conditionally replicating killer plasmids can be used. Such plasmids, which have been produced in accordance with the invention, can replicate in the donor but cannot replicate in the recipient bacterium simply because their cognate replication initiator protein (*e.g*., Rep) is produced in the former cells but not the latter cells. Another variant plasmid contains a temperature-sensitive mutation in the mentioned above *rep* gene, so it can replicate only at temperatures below 37°C. Hence, its replication will occur in bacteria applied on skin but it will not occur if such bacteria break into the body's core.

As used herein, the term "donor cell" refers to any of the above-listed cells, including dividing and non-dividing bacterial cells (minicells and maxicells), as well as conditionally non-functional cells.

The following examples are set forth to describe the invention in greater detail. They are intended to illustrate, not to limit, the invention. Unless otherwise specified, general cloning, microbiological, biochemical and molecular biological procedures such as those set forth in Sambrook et al., Molecular Cloning, Cold Spring Harbor Laboratory (1989) ("Sambrook et al.") or Ausubel et al. (eds) Current Protocols in Molecular Biology, John Wiley & Sons (2001) ("Ausubel et al.") are used.

### EXAMPLE 1

### Preparation of Runaway Replication Plasmid from Plasmid R6K

Plasmid R6K is an *Escherichia coli* conjugative plasmid believed to be a narrow host range. Replication of R6K derivatives containing its oriV, called y *ori,* generally requires a Rep protein, π, which is encoded by the plasmid's *pir* gene. The π protein is bifunctional in replication; it acts as an activator of replication at low cellular levels and an inhibitor of replication at elevated levels. For a review of R6K replication and its control by π protein, see Filutowicz & Rakowski (1998) Gene 223, 195-204.

Using site-directed mutagenesis, the inventor has obtained the following three types of mutations within the *pir* gene:
(1) double amino acid substitution: pro106leu, phe107ser (numbering of residues according to Stalker et al. (1982) J. Mol. Biol. 161: 33-43).
(2) deletion of codons 106 and 107; and
(3) deletion of codons 105, 106 and 107.

The γ *ori* was combined with the mutated *pir* genes in three configurations. In one configuration, the mutant gene was contained on a plasmid different from the plasmid containing the γ *ori*, thus providing π protein in *trans.* In another configuration, the mutant gene was integrated into the host chromosome, thus providing π protein also *in trans.* In third configuration the mutant *pir* gene was contained on the same plasmid with they *ori,* thus providing its function in *cis.*

### EXAMPLE 2

### Bacterial Cells Transformed with Plasmids Containing Mutated pir and ori in cis are Killed

*Escherichia coli* cells were transformed with either (1) the plasmids containing a mutated *pir* gene and the y *ori* in *trans*; or (2) a plasmid containing a mutated *pir* gene and the γ *ori* in *cis.*

In transformed cells containing the mutant *pir* and the γ *ori* in *trans,* the copy number of the γ *ori* plasmid was increased 20- to 25-fold in comparison to wild-type *pir* controls. Cells transformed with the mutant *pir* and the γ *ori* in *cis* were killed by the runaway replication of γ *ori.* The occurrence of the runaway phenotype when mutant *pir* is in *cis* to the *ori* but not in *trans* is believed to be caused by the enhanced effect of the origin activation and translation of nascent π protein occurring next to each other.

### EXAMPLE 3

### Preparation of Runaway Replication Plasmid from Plasmid RK2

Plasmid RK2 is a promiscuous plasmid that can replicate in 29 (and probably many more) gram-negative species (Guiney and Lanka, 1989, p 27-54. In C. M. Thomas (ed) Promiscous plasmids in gram-negative bacteria. London, Ltd London United Kingdom.). Plasmid RK2 is a 60-kb self-transmissible plasmid with a complete nucleotide sequence known (Pansegrau et al., 1994,. J. Mol. Biol. 239, 623-663). A minimal replicon derived from this large plasmid has been obtained that is devoid of all its genes except for a *trf*A gene, that encodes plasmid's Rep protein called TrfA, and an origin of vegetative replication *ori*V For a review of RK2 replication and its control by TrfA protein, see Helinski et al., 1996 (In Escherichia coli and Salmonella Cellular and Molecular Biology, Vol. 2 (ed. F. Neidhardt, et al., 2295-2324, ASM Press, Washington D.C.). Combinations of specific mutations in the *rep* gene of plasmid RK2 (*trf*A) confer run-away replication on the minimal, self-replicating plasmid derivatives (Haugan et al., 1995, Plasmid 33, 27-39; Toukdarian and Helinski, 1998, Gene 223, 205-211). Such plasmids elicit a killing effect when introduced into wild type *E*. *coli* strains by transformation or electroporation. The inventors laboratory also constructed a plasmid which can inflict killing on bacterial host conditionally. This was achieved by using an inducible promoter which governs expression of a hyperactive version of *trfA* (trfA264 267); in the absence of an inducer, plasmid copy number is low (harmless) but in the presence of the inducer run-away replication occures, killing the host. The run-away plasmids, both constitutive and conditional, can be maintained in specially constructed strains in which a wild-type allele of the *trf*A gene (providing replication repressor) is also present, thereby suppressing over-replication (killing) by complementation. This and the previous examples illustrate not only the use of R6K derivatives to kill unwanted bacteria (Example 2 above) but also specifically constructed derivatives of other plasmids such as RK2.

## Claims

1. A pharmaceutical preparation for use in treatment of a subject by reducing or eliminating a target population of bacteria in the subject, comprising an antimicrobial agent, which comprises a donor bacterial cell harboring at least one transmissible plasmid comprising:
a) an origin of replication for synthesizing the plasmid in the donor bacterial cell, wherein initiation of replication at the origin is negatively controlled by a plasmid replication repressor, wherein in the absence of the plasmid replication repressor, the transmissible plasmid undergoes runaway replication;
b) an origin of transfer from which conjugative transfer of the transmissible plasmid initiates from the donor cell to at least one recipient cell; and, optionally,
c) at least one selectable marker gene;
wherein the donor bacterial cell further comprises on its chromosome, on the transmissible plasmid or both, one or more transfer genes conferring upon the donor cell the ability to conjugatively transfer the transmissible plasmid to the recipient cell, and wherein the donor bacterial cell produces the plasmid replication repressor, and further wherein the at least one recipient cell does not produce the plasmid replication repressor, thereby enabling the transmissible plasmid to undergo runaway replication in the recipient cell to kill the recipient cell.

2. The pharmaceutical preparation of claim 1, wherein the plasmid replication repressor is a plasmid replication protein that comprises a copy number control function and the donor cell comprises a wild type gene for said plasmid replication protein while the transmissible plasmid comprises a mutated gene for said plasmid replication protein wherein the copy number control function of the protein product of the mutated gene is reduced in comparison to the wild type plasmid replication protein.

3. The pharmaceutical preparation of claim 2, wherein said plasmid replication protein is a pir gene from plasmid R6K, wherein said pir gene encodes a π protein.

4. The pharmaceutical preparation of claim 1, wherein said transmissible plasmid comprises a derivative from a transmissible plasmid selected from the group consisting of RK2, R6K, pCU1, p15A, pIP501, pAMβ1 and pCRG1600.

5. The pharmaceutical preparation of claim 2, wherein said transmissible plasmid comprises a derivative from plasmid R6K and the mutated gene for the plasmid replication protein comprises a mutation in the R6K pir gene such that its encoded protein comprises at least one amino acid deletion or substitution at amino acid 105, 106 or 107.

6. A pharmaceutical preparation for use in treatment of a subject by reducing or eliminating a target population of bacteria in the subject, comprising an antimicrobial agent, which comprises a donor bacterial cell harboring at least one transmissible plasmid comprising:
a) an origin of replication for synthesizing the plasmid in the donor bacterial cell;
b) an origin of transfer from which conjugative transfer of the transmissible plasmid initiates from the donor bacterial cell to at least one recipient cell;
c) at least one killer gene that, upon expression in a bacterial cell, produces a product that kills the cell; and, optionally,
d) at least one selectable marker gene;
wherein the donor bacterial cell further comprises on its chromosome, on the transmissible plasmid or both, one or more transfer genes conferring upon the bacterial donor cell the ability to conjugatively transfer the transmissible plasmid to the recipient cell, and wherein the donor bacterial cell is modified so as to be unaffected by the product of the killer gene, and further wherein the at least one recipient cell has not been modified so as to be unaffected by the product of the killer gene.

7. The pharmaceutical preparation of claim 3 or claim 6, wherein the transfer genes are contained on the transmissible plasmid, such that the transmissible plasmid is self-transmissible from the donor bacterial cell to a recipient cell, and further from the recipient cell to another recipient cell.

8. The pharmaceutical preparation of claim 6, wherein the killer gene kills the cells by being expressed and thereby producing a gene product that is detrimental or lethal to bacterial cells, and the donor bacterial cells have been modified so as to repress the expression of the killer gene, thereby avoiding production of the detrimental or lethal gene product.

9. The pharmaceutical preparation of claim 6, wherein the killer gene is obtained from a bacteriophage.

10. The pharmaceutical preparation of claim 9, wherein the bacteriophage is selected from the group consisting of T-series phages, P1, p22 and λ.

11. The pharmaceutical preparation of claim 2 or claim 6, wherein the donor bacterial cell is selected from the group consisting of dividing bacteria, maxicells, minicells and conditionally non-functional bacteria.

12. The pharmaceutical preparation of claim 11, wherein the donor bacterial cell is a non- pathogenic strain of bacteria selected from the group consisting of *Escherichia coli, Lactobacillus spp., Lactococcus, Bifidobacteria, Eubacteria,* and bacterial minicells.

13. The pharmaceutical preparation of claim 2 or 6, wherein the recipient cell is a pathogenic strain of bacterium selected from the group *Campylobacter spp., Enterobacter spp., Enterococcus spp., Escherichia coli, Gardnerella vaginalis, Haemophilus spp., Helicobacter pylorii, Mycobacterium tuberculosis, Propionobacter acnes, Pseudomonas aeruginosa* and other *Pseudomonas spp., Salmonella typhimurium, Shigella spp.* and *Staphylococcus spp*.

14. The pharmaceutical preparation of claim 2 or claim 6, wherein the origin of replication is derived from a plasmid selected from the group consisting of R6K, RK2, rts1, p15A and RSF1010.

15. The pharmaceutical preparation of claim 2 or claim 6, wherein the origin of replication is selected from the group consisting of F and P1.

16. The pharmaceutical preparation of claim 2 or claim 6, wherein the selectable marker gene confers a nutritional selection advantage on cells containing the transmissible plasmid.

17. The pharmaceutical preparation of claim 2 or claim 6, wherein the transfer genes are derived from a plasmid selected from the group consisting of F, R6K and Ti.

18. A pharmaceutical preparation of claim 1, claim 2 or claim 6 for reducing or eliminating a target population of bacteria in a subject, wherein the antimicrobial agent is formulated for a pre-determined route of administration to the subject.

19. The pharmaceutical preparation of claim 18, wherein the pre- determined route of administration is selected from the group consisting of: topical, oral, nasal, pulmonary, ophthalmic, aural, rectal, urogenital, subcutaneous, intraperitoneal and intravenous.

20. An in vitro method for reducing bacterial contamination, comprising: exposing said recipient cell to a donor bacterial cell that comprises:
a) a recombinant transmissible plasmid configured to undergo runaway lethal replication in said recipient cell wherein said transmissible plasmid comprises an origin of replication for synthesizing the plasmid in the donor bacterial cell, wherein initiation of replication at the origin is negatively controlled by a plasmid replication repressor, wherein in the absence of the plasmid replication repressor, the transmissible plasmid undergoes runaway replication; and
b) one or more transfer genes on the donor bacterial cell's chromosome, said transmissible plasmid, or both to confer upon said donor bacterial cell the ability to conjugatively transfer said transmissible plasmid to said recipient cell under conditions wherein said transmissible plasmid is conjugatively transferred from said donor bacterial cell to said recipient bacterial cell and said transmissible plasmid undergoes runaway replication in said recipient cell.

21. A method for reducing or eliminating a target population of bacteria in a plant comprising: exposing said recipient cell to a donor bacterial cell that comprises
a) a recombinant transmissible plasmid configured to undergo lethal runaway replication in said recipient cell wherein said transmissible plasmid comprises an origin of replication for synthesizing the plasmid in the donor bacterial cell, wherein initiation of replication at the origin is negatively controlled by a plasmid replication repressor, wherein in the absence of the plasmid replication repressor, the transmissible plasmid undergoes runaway replication; and
b) one or more transfer genes on the donor bacterial cell's chromosome, said transmissible plasmid, or both to confer upon said donor bacterial cell the ability to conjugatively transfer said transmissible plasmid to said recipient cell under conditions wherein said transmissible plasmid is conjugatively transferred from said donor bacterial cell to said recipient bacterial cell and said transmissible plasmid under goes runaway replication in said recipient cell, wherein said recipient cell resides in a plant.

22. The in vitro method of claim 20 or the method of claim 21, wherein said recombinant transmissible plasmid comprises an origin of transfer.

23. The in vitro method of claim 20 or the method of claim 21, wherein said recombinant transmissible plasmid comprises a portion of a plasmid selected from the group consisting of RK2, R6K, pCU1, p15A, pIP501, pAMβ1 and pCRG1600.

24. The method of claim 20 or claim 21, wherein said donor bacterial cell comprises a gene encoding a plasmid replication repressor.

25. The method of claim 24, wherein said recombinant transmissible plasmid comprises an origin of replication that is negatively controlled by said plasmid replication repressor.

26. The method of claim 25, wherein said origin of replication of said recombinant transmissible plasmid is from a plasmid selected from the group consisting of R6K, RK2, rts1, p15A, RSF1010, F, and P1.

27. The method of claim 24, wherein said gene encoding a plasmid replication repressor comprises a pir gene from plasmid R6K, wherein said pir gene encodes a π protein.

28. The method of claim 27, wherein said recombinant transmissible plasmid further comprises a gene encoding a mutant variant of said pir gene from plasmid R6K, wherein said mutant variant of a bifunctional protein is a mutant variant of a π protein.

29. The method of claim 28, wherein said mutant variant of a π protein comprises at least one amino acid deletion or substitution relative to the amino acid sequence of a non-mutant π protein.

30. The method of claim 29, wherein said at least one amino acid deletion or substitution comprises a deletion or substitution at amino acid 105, 106 or 107 of the amino acid sequence of a non-mutant π protein.

31. The method of claim 29, wherein said at least one amino acid deletion or substitution comprises a deletion or substitution at amino acids 106 and 107 of the amino acid sequence of a non-mutant π protein.

32. The in vitro method of claim 20 or the method of claim 21, wherein said donor bacterial cell is non-pathogenic.

33. The in vitro method of claim 20 or the method of claim 21, wherein said donor bacterial cell is selected from the group consisting of *Escherichia coli, Lactobacillus spp., Lactococcus, Bifidobacteria,* Eubacteria, and bacterial minicells.

34. The in vitro method of claim 31 or the method of claim 32, wherein said recipient cell is a pathogenic bacterium; or wherein said recipient cell is Gram-negative; or wherein said recipient cell is Gram-positive.

35. The in vitro method of claim 20 or the method of claim 21, wherein said recipient cell is a selected from the group consisting of *Campylobacter spp., Enterobacter spp., Enterococcus spp., Escherichia coli, Gardnerella vaginalis, Haemophilis spp., Helicobacter pylori, Mycobacterium tuberculosis, Propionobacter acnes, Pseudomonas aeruginosa* and other *Pseudomonas spp., Salmonella typhimurium, Shigella spp.* and *Staphylococcus spp.*

36. The in vitro method of claim 20 or the method of claim 21, wherein the method comprises the step of treating said recipient cell with the pharmaceutical preparation of claim 1 under conditions wherein said transmissible plasmid is conjugatively transferred from said donor cell to said recipient cell.

37. The in vitro method of claim 20 or the method of claim 21, wherein the method comprises the step of treating said recipient cell with the pharmaceutical preparation of claim 2 under conditions wherein said transmissible plasmid is conjugatively transferred from said donor cell to said recipient cell.

38. The in vitro method of claim 20 or the method of claim 21, wherein the recipient cell resides in a food or feed item.

39. An in vitro method of controlling bacterial contamination, comprising treating said recipient bacterial cell with an antimicrobial agent, which comprises a donor bacterial cell harboring at least one transmissible plasmid comprising:
a) an origin of replication for synthesizing the plasmid in the donor bacterial cell;
b) an origin of transfer from which conjugative transfer of the transmissible plasmid initiates from the donor bacterial cell to at least one recipient cell;
c) at least one killer gene that, upon expression in a bacterial cell, produces a product that kills the cell; and, optionally,
d) at least one selectable marker gene;
wherein the donor bacterial cell further comprises on its chromosome, on the transmissible plasmid or both, one or more transfer genes conferring upon the donor bacterial cell the ability to conjugatively transfer the transmissible plasmid to the recipient cell, and wherein the donor bacterial cell is modified so as to be unaffected by the product of the killer gene, and further wherein the at least one recipient cell has not been modified so as to be unaffected by the product of the killer gene, under conditions wherein said transmissible plasmid is conjugatively transferred from said donor bacterial cell to said recipient bacterial cell, and wherein said killer gene is expressed in said recipient bacterial cell.

40. A method of reducing or eliminating a target population of bacteria in a plant, comprising treating said recipient bacterial cell with an antimicrobial agent, which comprises a donor bacterial cell harboring at least one transmissible plasmid comprising:
a) an origin of replication for synthesizing the plasmid in the donor bacterial cell;
b) an origin of transfer from which conjugative transfer of the transmissible plasmid initiates from the donor bacterial cell to at least one recipient cell;
c) at least one killer gene that, upon expression in a bacterial cell, produces a product that kills the cell; and, optionally,
d) at least one selectable marker gene;
wherein the donor bacterial cell further comprises on its chromosome, on the transmissible plasmid or both, one or more transfer genes conferring upon the donor cell the ability to conjugatively transfer the transmissible plasmid to the recipient cell, and wherein the donor bacterial cell is modified so as to be unaffected by the product of the killer gene, and further wherein the at least one recipient cell has not been modified so as to be unaffected by the product of the killer gene, under conditions wherein said transmissible plasmid is conjugatively transferred from said donor bacterial cell to said recipient bacterial cell, and wherein said killer gene is expressed in said recipient bacterial cell, wherein the recipient cell resides in a plant.

41. The in vitro method of claim 39 or the method of claim 40, wherein said donor cell represses expression of said at least one killer gene.

42. The in vitro method of claim 39 or the method of claim 40, wherein said at least one killer gene comprises a gene of a bacteriophage.

43. The method of claim 42, wherein said bacteriophage is selected from the group consisting of T-series phages, P1, p22 and λ.

44. The in vitro method of claim 20 or claim 39 or the method of claim 21 or claim 40, wherein said one or more transfer genes are contained on said recombinant transmissible plasmid.

45. The in vitro method of claim 20 or claim 39 or the method of claim 21 or claim 40, wherein said one or more transfer genes are transfer genes of a plasmid selected from the group consisting of F, R6K and Ti.

46. The in vitro method of claim 39 or the method of claim 40, wherein said donor bacterial cell is non-pathogenic.

47. The in vitro method of claim 39 or the method of claim 40, wherein said donor bacterial cell is selected from the group consisting of *Escherichia coli, Lactobacillus spp., Lactococcus, Bifidobacteria, Eubacteria,* and bacterial minicells.

48. The in vitro method of claim 39 or the method of claim 40, wherein said recipient bacterial cell is a pathogenic bacterium; or wherein said recipient bacterial cell is Gram-negative; or wherein said recipient bacterial cell is Gram-positive.

49. The in vitro method of claim 39 or the method of claim 40, wherein said recipient bacterial cell is a selected from the group consisting of *Campylobacter spp., Enterobacter spp., Enterococcus spp., Escherichia coli, Gardnerella vaginalis, Haemophilis spp., Helicobacter pylori, Mycobacterium tuberculosis, Propionobacter acnes, Pseudomonas aeruginosa* and other *Pseudomonas spp., Salmonella typhimurium, Shigella spp.* and *Staphylococcus spp*.

50. The in vitro method of claim 39, wherein the recipient cell resides in a food or feed item.

51. A donor bacterial cell that comprises:
a) recombinant transmissible plasmid configured to undergo lethal runaway replication in a recipient cell wherein said transmissible plasmid comprises an origin of replication for synthesizing the plasmid in the donor cell, wherein initiation of replication at the origin is negatively controlled by a plasmid replication repressor, wherein in the absence of the plasmid replication repressor, the transmissible plasmid undergoes runaway replication; and
b) one or more transfer genes on the donor cell's chromosome, said transmissible plasmid or both to confer upon said donor cell the ability to conjugatively transfer said transmissible plasmid to said recipient cell, for use in a method to reduce or eliminate a target population of bacteria in a subject.

52. An antimicrobial agent according to claim 6 for use in a method of killing a recipient bacterial cell in a human or animal.

## Patentansprüche

1. Pharmazeutisches Präparat zur Verwendung bei der Behandlung eines Individuums durch Reduktion oder Beseitigung einer Zielpopulation von Bakterien in dem Individuum, wobei das Präparat ein antimikrobielles Mittel umfasst, das eine Donor-Bakterienzelle mit zumindest einem übertragbaren Plasmid aufweist, umfassend:
a) einen Replikationsursprung zur Synthese des Plasmids in der Donor-Bakterienzelle, wobei der Start der Replikation am Ursprung durch einen Plasmid-Replikationsrepressor negativ gesteuert wird, wobei das übertragbare Plasmid in Abwesenheit des Plasmid-Replikationsrepressors eine unkontrollierte Replikation durchläuft;
b) einen Transferursprung, von dem aus ein konjugativer Transfer des übertragbaren Plasmids von der Donorzelle zu zumindest einer Rezipientenzelle startet; und gegebenenfalls
c) zumindest ein selektierbares Markergen;
wobei die Donor-Bakterienzelle weiters auf ihrem Chromosom, auf dem übertragbaren Plasmid oder auf beiden ein oder mehrere Transfergene umfasst, die der Donorzelle die Fähigkeit verleihen, das übertragbare Plasmid konjugativ zur Rezipientenzelle zu übertragen, und wobei die Donor-Bakterienzelle den Plasmid-Replikationsrepressor erzeugt und wobei weiters die zumindest eine Rezipientenzelle den Plasmidreplikationsrepressor nicht erzeugt, wodurch es dem übertragbaren Plasmid ermöglicht wird, eine unkontrollierte Replikation in der Rezipientenzelle zu durchlaufen und so die Rezipientenzelle zu töten.

2. Pharmazeutisches Präparat nach Anspruch 1, worin der Plasmidreplikationsrepressor ein Plasmidreplikationsprotein ist, das eine Kopienzahl-Kontrollfunktion ausübt, und die Donorzelle ein Wildtyp-Gen für dieses Plasmidreplikationsprotein umfasst, während das übertragbare Plasmid ein mutiertes Gen für dieses Plasmidreplikationsprotein umfasst, wobei die Kopienzahl-Kontrollfunktion des Proteinprodukts des mutierten Gens im Vergleich zum Wildtyp-Plasmidreplikationsgen reduziert ist.

3. Pharmazeutisches Präparat nach Anspruch 2, worin das Plasmidreplikationsprotein ein pir-Gen vom Plasmid R6K ist, wobei das pir-Gen für ein π-Protein kodiert.

4. Pharmazeutisches Präparat nach Anspruch 1, worin das übertragbare Plasmid ein Derivat von einem übertragbaren Plasmid umfasst, das aus der aus RK2, R6K, pCU1, p15A, pIP501, pAMβ1 und pCRG1600 bestehenden Gruppe ausgewählt ist.

5. Pharmazeutisches Präparat nach Anspruch 2, worin das übertragbare Plasmid ein Derivat vom Plasmid R6K umfasst und das mutierte Gen für das Plasmidreplikationsprotein eine Mutation im R6K-pir-Gen umfasst, sodass sein kodiertes Protein zumindest eine Aminosäuredeletion oder -substitution an Aminosäure 105, 106 oder 107 umfasst.

6. Pharmazeutisches Präparat zur Verwendung bei der Behandlung eines Individuums durch Reduktion oder Beseitigung einer Zielpopulation von Bakterien in dem Individuum, wobei das Präparat ein antimikrobielles Mittel umfasst, das eine Donor-Bakterienzelle mit zumindest einem übertragbaren Plasmid aufweist, umfassend:
a) einen Replikationsursprung zur Synthese des Plasmids in der Donor-Bakterienzelle;
b) einen Transferursprung, von dem aus ein konjugativer Transfer des übertragbaren Plasmids von der Donor-Bakterienzelle zu zumindest einer Rezipientenzelle startet;
c) zumindest ein Killergen, das bei Expression in einer Bakterienzelle ein Produkt erzeugt, das die Zelle tötet, und gegebenenfalls
d) zumindest ein selektierbares Markergen;
wobei die Donor-Bakterienzelle weiters auf ihrem Chromosom, auf dem übertragbaren Plasmid oder auf beiden ein oder mehrere Transfergene umfasst, die der Bakterien-Donorzelle die Fähigkeit verleihen, das übertragbare Plasmid konjugativ zur Rezipientenzelle zu übertragen, und wobei die Donor-Bakterienzelle so modifiziert ist, dass sie vom Produkt des Killergens unbeeinflusst bleibt, und wobei weiters die zumindest eine Rezipientenzelle nicht so modifiziert ist, dass sie vom Produkt des Killergens unbeeinflusst bleibt.

7. Pharmazeutisches Präparat nach Anspruch 3 oder Anspruch 6, worin die Transfergene auf dem übertragbaren Plasmid vorhanden sind, sodass das übertragbare Plasmid sich selbst von der Donor-Bakterienzelle zu einer Rezipientenzelle und weiters von der Rezipientenzelle zu einer weiteren Rezipientenzelle übertragen kann.

8. Pharmazeutisches Präparat nach Anspruch 6, worin das Killergen die Zellen tötet, indem es exprimiert wird und dadurch ein Genprodukt erzeugt, das für Bakterienzellen schädlich oder tödlich ist, und die Donor-Bakterienzellen so modifiziert sind, dass sie die Expression des Killergens unterdrücken, wodurch die Produktion des schädlichen oder tödlichen Genprodukts verhindert wird.

9. Pharmazeutisches Präparat nach Anspruch 6, worin das Killergen von einem Bakteriophagen stammt.

10. Pharmazeutisches Präparat nach Anspruch 9, worin der Bakteriophage aus der aus Phagen der T-Reihe, P1, p22 und λ bestehenden Gruppe ausgewählt ist.

11. Pharmazeutisches Präparat nach Anspruch 2 oder Anspruch 6, worin die Donor-Bakterienzelle aus der aus sich teilenden Bakterien, Maxizellen, Minizellen und bedingungsabhängig nichtfunktionellen Bakterien bestehenden Gruppe ausgewählt ist.

12. Pharmazeutisches Präparat nach Anspruch 11, worin die Donor-Bakterienzelle ein nichtpathogener Stamm einer Bakterie ist, die aus der aus *Escherichia coli, Lactobacillus spp., Lactococcus, Bifidobakterien, Eubakterien* und bakteriellen Minizellen bestehenden Gruppe ausgewählt ist.

13. Pharmazeutisches Präparat nach Anspruch 2 oder Anspruch 6, worin die Rezipientenzelle ein pathogener Stamm einer Bakterie ist, die aus der aus *Campylobacter spp., Enterobacter spp., Enterococcus spp., Escherichia coli, Gardnerella vaginalis, Haemophilus spp., Helicobacter pylori, Mycobacterium tuberculosis, Propionobacter acnes, Pseudomonas aeruginosa* und anderen *Pseudomonas spp., Salmonella typhimurium, Shigella spp.* und *Staphylococcus spp.* bestehenden Gruppe ausgewählt ist.

14. Pharmazeutisches Präparat nach Anspruch 2 oder Anspruch 6, worin der Replikationsursprung von einem Plasmid abgeleitet ist, das aus der aus R6K, RK2, rts1, p15A und RSF1010 bestehenden Gruppe ausgewählt ist.

15. Pharmazeutisches Präparat nach Anspruch 2 oder Anspruch 6, worin der Replikationsursprung aus der aus F und P1 bestehenden Gruppe ausgewählt ist.

16. Pharmazeutisches Präparat nach Anspruch 2 oder Anspruch 6, worin das selektierbare Markergen Zellen, die das übertragbare Plasmid enthalten, einen Nahrungsselektionsvorteil verleihen.

17. Pharmazeutisches Präparat nach Anspruch 2 oder Anspruch 6, worin die Transfergene von einem Plasmid abgeleitet sind, das aus der aus F, R6K und Ti bestehenden Gruppe ausgewählt ist.

18. Pharmazeutisches Präparat nach Anspruch 1, Anspruch 2 oder Anspruch 6 zur Reduktion oder Beseitigung einer Zielpopulation von Bakterien in einem Individuum, wobei das antimikrobielle Mittel für einen vorbestimmten Verabreichungsweg an das Individuum formuliert ist.

19. Pharmazeutisches Präparat nach Anspruch 18, worin der vorbestimmte Verabreichungsweg aus folgender Gruppe ausgewählt ist: topisch, oral, nasal, pulmonal, ophthalmisch, aural, rektal, urogenital, subkutan, intraperitoneal und intravenös.

20. In-vitro-Verfahren zur Reduktion von Bakterienbefall, umfassend: das Aussetzen der Rezipientenzelle gegenüber einer Donor-Bakterienzelle, die Folgendes umfasst:
a) ein rekombinantes übertragbares Plasmid, das so konfiguriert ist, dass es in der Rezipientenzelle eine unkontrollierte tödliche Replikation durchläuft, wobei das übertragbare Plasmid einen Replikationsursprung zur Synthese des Plasmids in der Donor-Bakterienzelle umfasst, wobei der Start der Replikation am Ursprung durch einen Plasmid-Replikationsrepressor negativ gesteuert wird, wobei das übertragbare Plasmid in Abwesenheit des Plasmid-Replikationsrepressors eine unkontrollierte Replikation durchläuft;
b) ein oder mehrere Transfergene auf dem Chromosom der Donor-Bakterienzelle, auf dem übertragbaren Plasmid oder auf beiden, um der Donor-Bakterienzelle die Fähigkeit zu verleihen, das übertragbare Plasmid unter Bedingungen konjugativ zur Rezipientenzelle zu übertragen, unter denen das übertragbare Plasmid konjugativ von der Donor-Bakterienzelle zur Rezipienten-Bakterienzelle übertragen wird und das übertragbare Plasmid eine unkontrollierte Replikation in der Rezipientenzelle durchläuft.

21. Verfahren zur Reduktion oder Beseitigung einer Zielpopulation von Bakterien in einer Pflanze, das Folgendes umfasst: das Aussetzen einer Rezipientenzelle gegenüber einer Donor-Bakterienzelle, die Folgendes umfasst:
a) ein rekombinantes übertragbares Plasmid, das so konfiguriert ist, dass es in der Rezipientenzelle eine unkontrollierte tödliche Replikation durchläuft, wobei das übertragbare Plasmid einen Replikationsursprung zur Synthese des Plasmids in der Donor-Bakterienzelle umfasst, wobei der Start der Replikation am Ursprung durch einen Plasmid-Replikationsrepressor negativ gesteuert wird, wobei das übertragbare Plasmid in Abwesenheit des Plasmid-Replikationsrepressors eine unkontrollierte Replikation durchläuft; und
b) ein oder mehrere Transfergene auf dem Chromosom der Donor-Bakterienzelle, auf dem übertragbaren Plasmid oder auf beiden, um der Donor-Bakterienzelle die Fähigkeit zu verleihen, das übertragbare Plasmid unter Bedingungen konjugativ zur Rezipientenzelle zu übertragen, unter denen das übertragbare Plasmid konjugativ von der Donor-Bakterienzelle zur Rezipienten-Bakterienzelle übertragen wird und das übertragbare Plasmid eine unkontrollierte Replikation in der Rezipientenzelle durchläuft, wobei sich die Rezipientenzelle in einer Pflanze befindet.

22. In-vitro-Verfahren nach Anspruch 20 oder Verfahren nach Anspruch 21, worin das rekombinante übertragbare Plasmid einen Transferursprung umfasst.

23. In-vitro-Verfahren nach Anspruch 20 oder Verfahren nach Anspruch 21, worin das rekombinante übertragbare Plasmid einen Abschnitt eines Plasmids umfasst, das aus der aus RK2, R6K, pCU1, p15A, pIP501, pAMβ1 und pCRG1600 bestehenden Gruppe ausgewählt ist.

24. Verfahren nach Anspruch 20 oder Anspruch 21, worin die Donor-Bakterienzelle ein Gen umfasst, das für einen Plasmidreplikationsrepressor kodiert.

25. Verfahren nach Anspruch 24, worin das rekombinante übertragbare Plasmid einen Replikationsursprung umfasst, der durch einen Plasmid-Replikationsrepressor negativ gesteuert wird.

26. Verfahren nach Anspruch 25, worin der Replikationsursprung des rekombinanten übertragbaren Plasmids von einem Plasmid stammt, das aus der aus R6K, RK2, rts1, p15A, RSF1010, F und P1 bestehenden Gruppe ausgewählt ist.

27. Verfahren nach Anspruch 24, worin das Gen, das für einen Plasmidreplikationsrepressor kodiert, ein pir-Gen vom Plasmid R6K umfasst, wobei das pir-Gen für ein π-Protein kodiert.

28. Verfahren nach Anspruch 27, worin das rekombinante übertragbare Plasmid weiters ein Gen umfasst, das für eine mutierte Variante des pir-Gens vom Plasmid R6K kodiert, wobei die mutierte Variante eines bifunktionellen Proteins eine mutierte Variante eines π-Proteins ist.

29. Verfahren nach Anspruch 28, worin die mutierte Variante eines π-Proteins zumindest eine Aminosäuredeletion oder -substitution in Bezug auf die Aminosäuresequenz eines nichtmutierten π-Proteins umfasst.

30. Verfahren nach Anspruch 29, worin die zumindest eine Aminosäuredeletion oder -substitution eine Deletion oder Substitution an Aminosäure 105, 106 oder 107 der Aminosäuresequenz eines nichtmutierten π-Proteins umfasst.

31. Verfahren nach Anspruch 29, worin die zumindest eine Aminosäuredeletion oder -substitution eine Deletion oder Substitution an den Aminosäuren 106 und 107 der Aminosäuresequenz eines nichtmutierten π-Proteins umfasst.

32. In-vitro-Verfahren nach Anspruch 20 oder Verfahren nach Anspruch 21, worin die Donor-Bakterienzelle nichtpathogen ist.

33. In-vitro-Verfahren nach Anspruch 20 oder Verfahren nach Anspruch 21, worin die Donor-Bakterienzelle aus der aus *Escherichia coli, Lactobacillus spp., Lactococcus, Bifidobakterien, Eubakterien* und bakteriellen Minizellen bestehenden Gruppe ausgewählt ist.

34. In-vitro-Verfahren nach Anspruch 31 oder Verfahren nach Anspruch 32, worin die Rezipientenzelle eine pathogene Bakterie ist; oder worin die Rezipientenzelle gramnegativ ist; oder worin die Rezipientenzelle grampositiv ist.

35. In-vitro-Verfahren nach Anspruch 20 oder Verfahren nach Anspruch 21, worin die Rezipientenzelle aus der aus *Campylobacter spp., Enterobacter spp., Enterococcus spp., Escherichia coli, Gardnerella vaginalis, Haemophilus spp., Helicobacter pylori, Mycobacterium tuberculosis, Propionobacter acnes, Pseudomonas aeruginosa* und anderen *Pseudomonas spp., Salmonella typhimurium, Shigella spp.* und *Staphylococcus spp.* bestehenden Gruppe ausgewählt ist.

36. In-vitro-Verfahren nach Anspruch 20 oder Verfahren nach Anspruch 21, worin das Verfahren den Schritt des Behandelns der Rezipientenzelle mit einem pharmazeutischen Präparat nach Anspruch 1 unter Bedingungen umfasst, unter denen das übertragbare Plasmid konjugativ von der Donorzelle zu der Rezipientenzelle übertragen wird.

37. In-vitro-Verfahren nach Anspruch 20 oder Verfahren nach Anspruch 21, worin das Verfahren den Schritt des Behandelns der Rezipientenzelle mit einem pharmazeutischen Präparat nach Anspruch 2 unter Bedingungen umfasst, unter denen das übertragbare Plasmid konjugativ von der Donorzelle zu der Rezipientenzelle übertragen wird.

38. In-vitro-Verfahren nach Anspruch 20 oder Verfahren nach Anspruch 21, worin sich die Rezipientenzelle in einem Lebensmittel oder Futter befindet.

39. In-vitro-Verfahren zur Kontrolle von Bakterienbefall, welches das Behandeln der Rezipienten-Bakterienzelle mit einem antimikrobiellen Mittel umfasst, das eine Donor-Bakterienzelle mit zumindest einem übertragbaren Plasmid aufweist, umfassend:
a) einen Replikationsursprung zur Synthese des Plasmids in der Donor-Bakterienzelle;
b) einen Transferursprung, von dem aus ein konjugativer Transfer des übertragbaren Plasmids von der Donorzelle zu zumindest einer Rezipientenzelle startet;
c) zumindest ein Killergen, das bei Expression in einer Bakterienzelle ein Produkt erzeugt, das die Zelle tötet, und gegebenenfalls
d) zumindest ein selektierbares Markergen;
wobei die Donor-Bakterienzelle weiters auf ihrem Chromosom, auf dem übertragbaren Plasmid oder auf beiden ein oder mehrere Transfergene umfasst, die der Donor-Bakterienzelle die Fähigkeit verleihen, das übertragbare Plasmid konjugativ zur Rezipientenzelle zu übertragen, und wobei die Donor-Bakterienzelle so modifiziert ist, dass sie vom Produkt des Killergens unbeeinflusst bleibt, und wobei weiters die zumindest eine Rezipientenzelle nicht so modifiziert ist, dass sie vom Produkt des Killergens unbeeinflusst bleibt, unter Bedingungen, unter denen das übertragbare Plasmid konjugativ von der Donor-Bakterienzelle zu der Rezipienten-Bakterienzelle übertragen wird, und wobei das Killergen in der Rezipienten-Bakterienzelle exprimiert wird.

40. Verfahren zur Reduktion oder Beseitigung einer Zielpopulation von Bakterien in einer Pflanze, welches das Behandeln der Rezipienten-Bakterienzelle mit einem antimikrobiellen Mittel umfasst, das eine Donor-Bakterienzelle mit zumindest einem übertragbaren Plasmid aufweist, umfassend:
a) einen Replikationsursprung zur Synthese des Plasmids in der Donor-Bakterienzelle;
b) einen Transferursprung, von dem aus ein konjugativer Transfer des übertragbaren Plasmids von der Donor-Bakterienzelle zu zumindest einer Rezipientenzelle startet;
c) zumindest ein Killergen, das bei Expression in einer Bakterienzelle ein Produkt erzeugt, das die Zelle tötet, und gegebenenfalls
d) zumindest ein selektierbares Markergen;
wobei die Donor-Bakterienzelle weiters auf ihrem Chromosom, auf dem übertragbaren Plasmid oder auf beiden ein oder mehrere Transfergene umfasst, die der Donorzelle die Fähigkeit verleihen, das übertragbare Plasmid konjugativ zur Rezipientenzelle zu übertragen, und wobei die Donor-Bakterienzelle so modifiziert ist, dass sie vom Produkt des Killergens unbeeinflusst bleibt, und wobei weiters die zumindest eine Rezipientenzelle nicht so modifiziert ist, dass sie vom Produkt des Killergens unbeeinflusst bleibt, unter Bedingungen, unter denen das übertragbare Plasmid konjugativ von der Donor-Bakterienzelle zu der Rezipienten-Bakterienzelle übertragen wird, und wobei das Killergen in der Rezipienten-Bakterienzelle exprimiert wird, wobei sich die Rezipientenzelle in einer Pflanze befindet.

41. In-vitro-Verfahren nach Anspruch 39 oder Verfahren nach Anspruch 40, worin die Donorzelle die Expression des zumindest einen Killergens unterdrückt.

42. In-vitro-Verfahren nach Anspruch 39 oder Verfahren nach Anspruch 40, worin das zumindest eine Killergen ein Gen eines Bakteriophagen umfasst.

43. Verfahren nach Anspruch 42, worin der Bakteriophage aus der aus Phagen der T-Reihe, P1, p22 und λ bestehenden Gruppe ausgewählt ist.

44. In-vitro-Verfahren nach Anspruch 20 oder Anspruch 39 oder Verfahren nach Anspruch 21 oder Anspruch 40, worin das eine oder die mehreren Transfergene auf dem rekombinanten übertragbaren Plasmid vorhanden sind.

45. In-vitro-Verfahren nach Anspruch 20 oder Anspruch 39 oder Verfahren nach Anspruch 21 oder Anspruch 40, worin das eine oder die mehreren Transfergene Transfergene eines Plasmids sind, das aus der aus F, R6K und Ti bestehenden Gruppe ausgewählt ist.

46. In-vitro-Verfahren nach Anspruch 39 oder Verfahren nach Anspruch 40, worin die Donor-Bakterienzelle nichtpathogen ist.

47. In-vitro-Verfahren nach Anspruch 39 oder Verfahren nach Anspruch 40, worin die Donor-Bakterienzelle aus der aus *Escherichia coli, Lactobacillus spp., Lactococcus, Bifidobakterien, Eubakterien* und bakteriellen Minizellen bestehenden Gruppe ausgewählt ist.

48. In-vitro-Verfahren nach Anspruch 39 oder Verfahren nach Anspruch 40, worin die Rezipienten-Bakterienzelle eine pathogene Bakterie ist; oder worin die Rezipienten-Bakterienzelle gramnegativ ist; oder worin die Rezipienten-Bakterienzelle grampositiv ist.

49. In-vitro-Verfahren nach Anspruch 39 oder Verfahren nach Anspruch 40, worin die Rezipienten-Bakterienzelle aus der aus *Campylobacter spp., Enterobacter spp., Enterococcus spp., Escherichia coli, Gardnerella vaginalis, Haemophilus spp., Helicobacter pylori, Mycobacterium tuberculosis, Propionobacter acnes, Pseudomonas aeruginosa* und anderen *Pseudomonas spp., Salmonella typhimurium, Shigella spp.* und *Staphylococcus spp.* bestehenden Gruppe ausgewählt ist.

50. In-vitro-Verfahren nach Anspruch 39, worin sich die Rezipientenzelle in einem Lebensmittel oder Futter befindet.

51. Donor-Bakterienzelle, die Folgendes umfasst:
a) ein rekombinantes übertragbares Plasmid, das so konfiguriert ist, dass es in der Rezipientenzelle eine unkontrollierte tödliche Replikation durchläuft, wobei das übertragbare Plasmid einen Replikationsursprung zur Synthese des Plasmids in der Donorzelle umfasst, wobei der Start der Replikation am Ursprung durch einen Plasmid-Replikationsrepressor negativ gesteuert wird, wobei das übertragbare Plasmid in Abwesenheit des Plasmid-Replikationsrepressors eine unkontrollierte Replikation durchläuft; und
b) ein oder mehrere Transfergene auf dem Chromosom der Donorzelle, auf dem übertragbaren Plasmid oder auf beiden, um der Donorzelle die Fähigkeit zu verleihen, das übertragbare Plasmid konjugativ zur Rezipientenzelle zu übertragen, zur Verwendung in einem Verfahren zur Reduktion oder Beseitigung einer Zielpopulation von Bakterien in einem Individuum.

52. Antimikrobielles Mittel nach Anspruch 6 zur Verwendung in einem Verfahren zum Töten einer Rezipienten-Bakterienzelle in einem Menschen oder Tier.

## Revendications

1. Préparation pharmaceutique destinée à être utilisée dans le traitement d'un sujet en réduisant ou en éliminant une population cible de bactéries chez le sujet, comprenant un agent antimicrobien, qui comprend une cellule bactérienne donneuse abritant au moins un plasmide transmissible, comprenant:
a) une origine de réplication pour synthétiser le plasmide dans la cellule bactérienne donneuse, où l'initiation de la réplication au niveau de l'origine est négativement contrôlée par un répresseur de réplication plasmidique où, en l'absence du répresseur de réplication plasmidique, le plasmide transmissible subit une réplication non contrôlée;
b) une origine de transfert à partir de laquelle un transfert conjugatif du plasmide transmissible est initié à partir de la cellule donneuse vers au moins une cellule receveuse; et, facultativement,
c) au moins un gène marqueur de sélection;
où la cellule bactérienne donneuse comprend en outre sur son chromosome, sur le plasmide transmissible ou les deux, un ou plusieurs gènes de transfert qui confèrent à la cellule donneuse une aptitude à transférer de manière conjuguée le plasmide transmissible à la cellule receveuse, et où la cellule bactérienne donneuse produit le répresseur de réplication plasmidique, et où en outre la au moins une cellule receveuse ne produit pas le répresseur de réplication plasmidique, ce qui permet au plasmide transmissible de subir une réplication non contrôlée dans la cellule receveuse afin de tuer la cellule receveuse.

2. Préparation pharmaceutique selon la revendication 1, dans laquelle le répresseur de réplication plasmidique est une protéine de réplication plasmidique qui comprend une fonction de contrôle du nombre de copies et la cellule donneuse comprend un gène de type sauvage pour ladite protéine de réplication plasmidique alors que le plasmide transmissible comprend un gène muté pour ladite protéine de réplication plasmidique, où la fonction de contrôle du nombre de copies du produit protéique du gène muté est réduite par comparaison à la protéine de réplication plasmidique de type sauvage.

3. Préparation pharmaceutique selon la revendication 2, dans laquelle ladite protéine de réplication plasmidique est un gène pir du plasmide R6K, où ledit gène pir code pour une protéine n.

4. Préparation pharmaceutique selon la revendication 1, dans laquelle ledit plasmide transmissible comprend un dérivé d'un plasmide transmissible sélectionné dans le groupe consistant en RK2, R6K, pCU1, P15A, pIP501, pAMβ1 et pCRG1600.

5. Préparation pharmaceutique selon la revendication 2, dans laquelle ledit plasmide transmissible comprend un dérivé du plasmide R6K et le gène muté pour la protéine de réplication plasmidique comprend une mutation dans le gène pir de R6K de sorte que sa protéine codée comprenne au moins une délétion ou une substitution d'acide aminé à l'acide aminé 105, 106 ou 107.

6. Préparation pharmaceutique destinée à être utilisée dans le traitement d'un sujet en réduisant ou en éliminant une population cible de bactéries chez le sujet, comprenant un agent antimicrobien, qui comprend une cellule bactérienne donneuse abritant au moins un plasmide transmissible, comprenant:
a) une origine de réplication pour synthétiser le plasmide dans la cellule bactérienne donneuse;
b) une origine de transfert à partir de laquelle un transfert conjugatif du plasmide transmissible est initié à partir de la cellule bactérienne donneuse vers au moins une cellule receveuse;
c) au moins un gène tueur qui, suite à une expression dans une cellule bactérienne, génère un produit qui tue la cellule; et, facultativement,
d) au moins un gène marqueur de sélection;
où la cellule bactérienne donneuse comprend en outre sur son chromosome, sur le plasmide transmissible ou les deux, un ou plusieurs gènes de transfert qui confèrent à la cellule bactérienne donneuse une aptitude à transférer de manière conjuguée le plasmide transmissible vers la cellule receveuse, et où la cellule bactérienne donneuse est modifiée de sorte à ne pas être affectée par le produit du gène tueur, et où en outre la au moins une cellule receveuse n'a pas été modifiée de sorte à ne pas être affectée par le produit du gène tueur.

7. Préparation pharmaceutique selon la revendication 3 ou la revendication 6, dans laquelle les gènes de transfert sont contenus sur le plasmide transmissible, de sorte que le plasmide transmissible soit autotransmissible à partir de la cellule bactérienne donneuse vers une cellule receveuse, et en outre à partir de la cellule receveuse vers une autre cellule receveuse.

8. Préparation pharmaceutique selon la revendication 6, dans laquelle le gène tueur tue les cellules en étant exprimé et en générant ainsi un produit génique qui est nuisible ou létal pour les cellules bactériennes, et les cellules bactériennes donneuses ont été modifiées de sorte à réprimer l'expression du gène tueur, ce qui permet ainsi d'éviter la production du produit génique nuisible ou létal.

9. Préparation pharmaceutique selon la revendication 6, dans laquelle le gène tueur est obtenu à partir d'un bactériophage.

10. Préparation pharmaceutique selon la revendication 9, dans laquelle le bactériophage est sélectionné dans le groupe consistant en des phages de la série T, Pl, p22 et λ.

11. Préparation pharmaceutique selon la revendication 2 ou la revendication 6, dans laquelle la cellule bactérienne donneuse est sélectionnée dans le groupe consistant en des bactéries en division, des maxi-cellules, des mini-cellules et des bactéries non fonctionnelles sous certaines conditions.

12. Préparation pharmaceutique selon la revendication 11, dans laquelle la cellule bactérienne donneuse est une souche non pathogène de bactéries sélectionnées dans le groupe consistant en *Escherichia coli, l*'*espèce Lactobacillus, Lactococcus, Bifidobacteria, Eubacteria,* et des mini-cellules bactériennes.

13. Préparation pharmaceutique selon la revendication 2 ou 6, dans laquelle la cellule receveuse est une souche pathogène d'une bactérie sélectionnée dans le groupe consistant en *l'espèce Campylobacter, l'espèce Enterobacter, l'espèce Enterococcus, Escherichia coli*, *Gardnerella vaginalis, l'espèce Haemophilus, Helicobacter pylori, Mycobacterium tuberculosis, Propionobacter acnes, Pseudomonas aeruginosa* et d'autres *espèces Pseudomonas, Salmonella typhimurium, l'espèce Shigella* et *l'espèce Staphylococcus.*

14. Préparation pharmaceutique selon la revendication 2 ou la revendication 6, dans laquelle l'origine de réplication est dérivée d'un plasmide sélectionné dans le groupe consistant en R6K, RK2, rts1, p15A et RSF1010.

15. Préparation pharmaceutique selon la revendication 2 ou la revendication 6, dans laquelle l'origine de réplication est sélectionnée dans le groupe consistant en F et P1.

16. Préparation pharmaceutique selon la revendication 2 ou la revendication 6, dans laquelle le gène marqueur de sélection confère un avantage de sélection nutritionnelle aux cellules contenant le plasmide transmissible.

17. Préparation pharmaceutique selon la revendication 2 ou la revendication 6, dans laquelle les gènes de transfert sont dérivés d'un plasmide sélectionné dans le groupe consistant en F, R6K et Ti.

18. Préparation pharmaceutique selon la revendication 1, la revendication 2 ou la revendication 6, destinée à réduire ou à éliminer une population cible de bactéries chez un sujet, dans laquelle l'agent antimicrobien est formulé pour une voie d'administration prédéterminée au sujet.

19. Préparation pharmaceutique selon la revendication 18, dans laquelle la voie d'administration prédéterminée est sélectionnée dans le groupe consistant en: la voie topique, orale, nasale, pulmonaire, ophtalmique, auriculaire, rectale, urogénitale, sous-cutanée, intrapéritonéale et intraveineuse.

20. Procédé in vitro pour réduire une contamination bactérienne, consistant à: exposer ladite cellule receveuse à une cellule bactérienne donneuse qui comprend:
a) un plasmide transmissible recombinant configuré pour subir une réplication létale non contrôlée dans ladite cellule receveuse, où ledit plasmide transmissible comprend une origine de réplication afin de synthétiser le plasmide dans la cellule bactérienne donneuse, où l'initiation de la réplication au niveau de l'origine est négativement contrôlée par un répresseur de réplication plasmidique où, en l'absence du répresseur de réplication plasmidique, le plasmide transmissible subit une réplication non contrôlée; et
b) un ou plusieurs gènes de transfert sur le chromosome de la cellule bactérienne donneuse, ledit plasmide transmissible, ou les deux, afin de conférer à ladite cellule bactérienne donneuse une aptitude à transférer de manière conjuguée ledit plasmide transmissible vers ladite cellule receveuse dans des conditions où ledit plasmide transmissible est transféré de manière conjuguée à partir de ladite cellule bactérienne donneuse vers ladite cellule bactérienne receveuse et ledit plasmide transmissible subit une réplication non contrôlée dans ladite cellule receveuse.

21. Procédé pour réduire ou éliminer une population cible de bactéries dans une plante, consistant à: exposer ladite cellule receveuse à une cellule bactérienne donneuse qui comprend
a) un plasmide transmissible recombinant configuré pour subir une réplication létale non contrôlée dans ladite cellule receveuse, où ledit plasmide transmissible comprend une origine de réplication afin de synthétiser le plasmide dans la cellule bactérienne donneuse, où l'initiation de la réplication au niveau de l'origine est négativement contrôlée par un répresseur de réplication plasmidique où, en l'absence du répresseur de réplication plasmidique, le plasmide transmissible subit une réplication non contrôlée; et
b) un ou plusieurs gènes de transfert sur le chromosome de la cellule bactérienne donneuse, ledit plasmide transmissible, ou les deux, afin de conférer à ladite cellule bactérienne donneuse une aptitude à transférer de manière conjuguée ledit plasmide transmissible vers ladite cellule receveuse dans des conditions où ledit plasmide transmissible est transféré de manière conjuguée à partir de ladite cellule bactérienne donneuse vers ladite cellule bactérienne receveuse et ledit plasmide transmissible subit une réplication non contrôlée dans ladite cellule receveuse, où ladite cellule receveuse réside dans une plante.

22. Procédé *in vitro* selon la revendication 20 ou procédé selon la revendication 21, dans lequel ledit plasmide transmissible recombinant comprend une origine de transfert.

23. Procédé *in vitro* selon la revendication 20 ou procédé selon la revendication 21, dans lequel ledit plasmide transmissible recombinant comprend une partie d'un plasmide sélectionné dans le groupe consistant en RK2, R6K, pCU1, p15A, pIP501, pAMβ1 et pCRG1600.

24. Procédé selon la revendication 20 ou la revendication 21, dans lequel ladite cellule bactérienne donneuse comprend un gène codant pour un répresseur de réplication plasmidique.

25. Procédé selon la revendication 24, dans lequel ledit plasmide transmissible recombinant comprend une origine de réplication qui est négativement contrôlée par ledit répresseur de réplication plasmidique.

26. Procédé selon la revendication 25, dans lequel ladite origine de réplication dudit plasmide transmissible recombinant provient d'un plasmide sélectionné dans le groupe consistant en R6K, RK2, rts1, p15A, RSF1010, F, et P1.

27. Procédé selon la revendication 24, dans lequel ledit gène codant pour un répresseur de réplication plasmidique comprend un gène pir du plasmide R6K, où ledit gène pir code pour une protéine n.

28. Procédé selon la revendication 27, dans lequel ledit plasmide transmissible recombinant comprend en outre un gène codant pour un variant mutant dudit gène pir du plasmide R6K, où ledit variant mutant d'une protéine bifonctionnelle est un variant mutant d'une protéine n.

29. Procédé selon la revendication 28, dans lequel ledit variant mutant d'une protéine n comprend au moins une délétion ou substitution d'acide aminé par rapport à la séquence d'acides aminés d'une protéine n non mutante.

30. Procédé selon la revendication 29, dans lequel ladite au moins une délétion ou substitution d'acide aminé comprend une délétion ou une substitution à l'acide aminé 105, 106 ou 107 de la séquence d'acides aminés d'une protéine n non mutante.

31. Procédé selon la revendication 29, dans lequel ladite au moins une délétion ou substitution d'acide aminé comprend une délétion ou une substitution aux acides aminés 106 et 107 de la séquence d'acides aminés d'une protéine n non mutante.

32. Procédé *in vitro* selon la revendication 20 ou procédé selon la revendication 21, dans lequel ladite cellule bactérienne donneuse est non pathogène.

33. Procédé *in vitro* selon la revendication 20 ou procédé selon la revendication 21, dans lequel ladite cellule bactérienne donneuse est sélectionnée dans le groupe consistant en *Escherichia coli*, *l'espèce Lactobacillus, Lactococcus, Bifidobacteria, Eubacteria,* et des mini-cellules bactériennes.

34. Procédé *in vitro* selon la revendication 31 ou procédé selon la revendication 32, dans lequel ladite cellule receveuse est une bactérie pathogène; ou dans lequel ladite cellule receveuse est Gram-négative; ou dans lequel ladite cellule receveuse est Gram-positive.

35. Procédé *in vitro* selon la revendication 20 ou procédé selon la revendication 21, dans lequel ladite cellule receveuse est sélectionnée dans le groupe consistant en *l'espèce Campylobacter, l'espèce Enterobacter, l'espèce Enterococcus, Escherichia coli, Gardnerella vaginalis, l'espèce Haemophilus, Helicobacter pylori, Mycobacterium tuberculosis, Propionobacter acnes, Pseudomonas aeruginosa* et d'autres *espèces Pseudomonas, Salmonella typhimurium, l'espèce Shigella* et *l'espèce Staphylococcus.*

36. Procédé *in vitro* selon la revendication 20 ou procédé selon la revendication 21, où le procédé comprend l'étape qui consiste à traiter ladite cellule receveuse avec la préparation pharmaceutique selon la revendication 1, dans des conditions où ledit plasmide transmissible est transféré de manière conjuguée à partir de ladite cellule donneuse vers ladite cellule receveuse.

37. Procédé *in vitro* selon la revendication 20 ou procédé selon la revendication 21, où le procédé comprend l'étape qui consiste à traiter ladite cellule receveuse avec la préparation pharmaceutique selon la revendication 2, dans des conditions où ledit plasmide transmissible est transféré de manière conjuguée à partir de ladite cellule donneuse vers ladite cellule receveuse.

38. Procédé *in vitro* selon la revendication 20 ou procédé selon la revendication 21, dans lequel la cellule receveuse réside dans un aliment ou un article alimentaire.

39. Procédé *in vitro* pour contrôler une contamination bactérienne, qui consiste à traiter ladite cellule bactérienne receveuse avec un agent antimicrobien, qui comprend une cellule bactérienne donneuse abritant au moins un plasmide transmissible, comprenant:
a) une origine de réplication pour synthétiser le plasmide dans la cellule bactérienne donneuse;
b) une origine de transfert à partir de laquelle un transfert conjugatif du plasmide transmissible est initié à partir de la cellule bactérienne donneuse vers au moins une cellule receveuse;
c) au moins un gène tueur qui, suite à une expression dans une cellule bactérienne, génère un produit qui tue la cellule; et, facultativement,
d) au moins un gène marqueur de sélection;
où la cellule bactérienne donneuse comprend en outre sur son chromosome, sur le plasmide transmissible ou les deux, un ou plusieurs gènes de transfert qui confèrent à la cellule bactérienne donneuse une aptitude à transférer de manière conjuguée le plasmide transmissible vers la cellule receveuse, et où la cellule bactérienne donneuse est modifiée de sorte à ne pas être affectée par le produit du gène tueur, et où en outre la au moins une cellule receveuse n'a pas été modifiée de sorte à ne pas être affectée par le produit du gène tueur, dans des conditions où ledit plasmide transmissible est transféré de manière conjuguée à partir de ladite cellule bactérienne donneuse vers ladite cellule bactérienne receveuse, et où ledit gène tueur est exprimé dans ladite cellule bactérienne receveuse.

40. Procédé pour réduire ou éliminer une population cible de bactéries dans une plante, qui consiste à traiter ladite cellule bactérienne receveuse avec un agent antimicrobien, qui comprend une cellule bactérienne donneuse abritant au moins un plasmide transmissible, comprenant:
a) une origine de réplication pour synthétiser le plasmide dans la cellule bactérienne donneuse;
b) une origine de transfert à partir de laquelle un transfert conjugatif du plasmide transmissible est initié à partir de la cellule bactérienne donneuse vers au moins une cellule receveuse;
c) au moins un gène tueur qui, suite à une expression dans une cellule bactérienne, génère un produit qui tue la cellule; et, facultativement,
d) au moins un gène marqueur de sélection;
où la cellule bactérienne donneuse comprend en outre sur son chromosome, sur le plasmide transmissible ou les deux, un ou plusieurs gènes de transfert qui confèrent à la cellule donneuse une aptitude à transférer de manière conjuguée le plasmide transmissible vers la cellule receveuse, et où la cellule bactérienne donneuse est modifiée de sorte à ne pas être affectée par le produit du gène tueur, et où en outre la au moins une cellule receveuse n'a pas été modifiée de sorte à ne pas être affectée par le produit du gène tueur, dans des conditions où ledit plasmide transmissible est transféré de manière conjuguée à partir de ladite cellule bactérienne donneuse vers ladite cellule bactérienne receveuse, et où ledit gène tueur est exprimé dans ladite cellule bactérienne receveuse, où la cellule receveuse réside dans une plante.

41. Procédé *in vitro* selon la revendication 39 ou procédé selon la revendication 40, dans lequel ladite cellule donneuse réprime l'expression dudit au moins un gène tueur.

42. Procédé *in vitro* selon la revendication 39 ou procédé selon la revendication 40, dans lequel ledit au moins un gène tueur comprend un gène d'un bactériophage.

43. Procédé selon la revendication 42, dans lequel ledit bactériophage est sélectionné dans le groupe consistant en des phages de la série T, P1, p22 et λ.

44. Procédé *in vitro* selon la revendication 20 ou la revendication 39 ou procédé selon la revendication 21 ou la revendication 40, dans lequel lesdits un ou plusieurs gènes de transfert sont contenus sur ledit plasmide transmissible recombinant.

45. Procédé *in vitro* selon la revendication 20 ou la revendication 39 ou procédé selon la revendication 21 ou la revendication 40, dans lequel lesdits un ou plusieurs gènes de transfert sont des gènes de transfert d'un plasmide sélectionné dans le groupe consistant en F, R6K et Ti.

46. Procédé *in vitro* selon la revendication 39 ou procédé selon la revendication 40, dans lequel ladite cellule bactérienne donneuse est non pathogène.

47. Procédé in vitro selon la revendication 39 ou procédé selon la revendication 40, dans lequel ladite cellule bactérienne donneuse est sélectionnée dans le groupe consistant en *Escherichia* coli, *l'espèce Lactobacillus, Lactococcus, Bifidobacteria, Eubacteria,* et des mini-cellules bactériennes.

48. Procédé *in vitro* selon la revendication 39 ou procédé selon la revendication 40, dans lequel ladite cellule bactérienne receveuse est une bactérie pathogène; ou dans lequel ladite cellule bactérienne receveuse est Gram-négative; ou dans lequel ladite cellule bactérienne receveuse est Gram-positive.

49. Procédé *in vitro* selon la revendication 39 ou procédé selon la revendication 40, dans lequel ladite cellule bactérienne receveuse est sélectionnée dans le groupe consistant en *l'espèce Campylobacter, l'espèce Enterobacter, l'espèce Enterococcus, Escherichia coli, Gardnerella vaginalis, l'espèce Haemophilus, Helicobacter pylori, Mycobacterium tuberculosis, Propionobacter acnes, Pseudomonas aeruginosa* et d'autres *espèces Pseudomonas, Salmonella typhimurium, l'espèce Shigella* et *l'espèce Staphylococcus.*

50. Procédé in vitro selon la revendication 39, dans lequel la cellule receveuse réside dans un aliment ou un article alimentaire.

51. Cellule bactérienne donneuse qui comprend:
a) un plasmide transmissible recombinant configuré pour subir une réplication létale non contrôlée dans une cellule receveuse, où ledit plasmide transmissible comprend une origine de réplication afin de synthétiser le plasmide dans la cellule donneuse, où l'initiation de la réplication au niveau de l'origine est négativement contrôlée par un répresseur de réplication plasmidique où, en l'absence du répresseur de réplication plasmidique, le plasmide transmissible subit une réplication non contrôlée; et
b) un ou plusieurs gènes de transfert sur le chromosome de la cellule donneuse, ledit plasmide transmissible ou les deux, afin de conférer à ladite cellule donneuse une aptitude à transférer de manière conjuguée ledit plasmide transmissible vers ladite cellule receveuse, pour une utilisation dans un procédé destiné à réduire ou à éliminer une population cible de bactéries chez un sujet.

52. Agent antimicrobien selon la revendication 6, destiné à être utilisé dans un procédé pour tuer une cellule bactérienne receveuse chez un humain ou un animal.
